# EUROPEAN PATENT SPECIFICATION

(11) **EP 3 984 982 B1**
(45) Date of publication and mention of the grant of the patent: **25.11.2026**
(21) Application number: 20844453.9
(22) Date of filing: 17.07.2020
(51) Int. Cl.: C07B 35/02, C07D 471/08, C07D 471/22

(54) **METHOD FOR PRODUCING MORPHINAN DERIVATIVE**
VERFAHREN ZUR HERSTELLUNG EINES MORPHINAN-DERIVATS
PROCÉDÉ DE PRODUCTION D'UN DÉRIVÉ DE MORPHINANE

(30) Priority: 19.07.2019 JP 2019134051
(43) Date of publication of application: 20.04.2022
(73) Proprietor: Nippon Chemiphar Co., Ltd., Tokyo 101-0032 (JP)
(72) Inventor: SAITO, Daisuke, Misato-shi, Saitama 341-0005 (JP); HAYASHIDA, Kohei, Misato-shi, Saitama 341-0005 (JP)
(74) Representative: Godemeyer Blum Lenze Patentanwälte Partnerschaft mbB - werkpatent
(86) International application number: PCT/JP2020/027767
(87) International publication number: WO 2021/015108

(56) References cited:
- EP-A1- 2 774 926
- WO-A1-2013/035833
- WO-A1-2014/136305
- WO-A1-2016/148232
- JP-A- 2015 180 605
- US-A- 5 504 208
- HAYASHIDA KOHEI ET AL: "Novel delta opioid receptor agonists with oxazatricyclodecane structure showing potent agonistic activities", BIOORGANIC & MEDICINAL CHEMISTRY LETTERS, vol. 27, no. 12, 1 June 2017 (2017-06-01), Amsterdam NL, pages 2742 - 2745, XP055786181, ISSN: 0960-894X, DOI: 10.1016/j.bmcl.2017.04.059
- SAWA Y.K. ET AL: "Elimination of the 4-hydroxyl group of the alkaloids related to morphine?IV", TETRAHEDRON, vol. 20, no. 10, 1 January 1964 (1964-01-01), AMSTERDAM, NL, pages 2255 - 2258, XP055786425, ISSN: 0040-4020, DOI: 10.1016/S0040-4020(01)97612-4
- "Greene's Protective Groups in Organic Synthesis", vol. 12, 1 January 2014, JOHN WILEY & SONS PROQUEST EBOOK CENTRAL, article WUTS PETER G M ET AL: "Reactivity Charts 8-10. Protection for the Amino Group", pages: 1298 - 1309, XP093006347
- PIRKLE WILLIAM H. ET AL.: "The Derivation of Hydroaromatic Morphinans and Isomorphinans from Natural Sources", THE JOURNAL OF ORGANIC CHEMISTRY, vol. 30, no. 6, June 1965 (1965-06-01), pages 1769 - 1773, XP055786400
- LI FUYING ET AL.: "Synthesis and opioid receptor activity of indolopropellanes", BIOORGANIC & MEDICINAL CHEMISTRY LETTERS, vol. 19, 2009, pages 4603 - 4606, XP055169332, DOI: 10.1016/j.bmcl.2009.06.093
- LI FUYING ET AL.: "Synthesis and SAR Study of Opioid Receptor Ligands: Mono- and Bis-Indolomorphinans", CHEMICAL BIOLOGY & DRUG DESIGN, vol. 74, no. 4, October 2009 (2009-10-01), pages 335 - 342, XP055786403
- ZHANG AO ET AL.: "Synthesis and Pharmacological Evaluation of 6,7-Indolo/ Thiazolo-Morphinanss-Further SAR of Levorphanol", JOURNAL OF MEDICINAL CHEMISTRY, vol. 50, no. 11, 1 May 2007 (2007-05-01), pages 2747 - 2751, XP055093411
- SAWA Y. K. ET AL.: "Elimination of the 4-hydroxyl group of the alkaloids related to morphine—XI", TETRAHEDRON, vol. 24, no. 20, January 1968 (1968-01-01), pages 6185 - 6196, XP055786411
- SELFRIDGE BRANDON R. ET AL.: "Structure-Activity Relationships of (+)- Naltrexone-Inspired Toll-like Receptor 4 (TLR4) Antagonists", JOURNAL OF MEDICINAL CHEMISTRY, vol. 58, no. 12, 2015, pages 5038 - 5052, XP055341920, DOI: 10.1021/acs.jmedchem.5b00426
- LINDERS J. T. M. ET AL.: "Diels-Alder reaction of 6-demethoxy-j3- dihydrothebaine with methyl vinyl ketone using microwave heating; preparation and pharmacology of 3-hydroxy-a, a, 17-trimethyl -6 beta,14 beta - ethenomorphinan-7beta -methanol, a novel deoxygenated diprenorphine analogue (chemistry of opium alkaloids, part XX", RECUEIL DES TRAVAUX CHIMIQUES DES PAYS-BAS, vol. 107, no. 6, 1 June 1988 (1988-06-01), pages 449 - 454, XP055786416
- REVESZ LASZLO ET AL.: "Design and Synthesis of Novel Opiate Antagonists with LH-Stimulating Properties", HELVETICA CHIMICA ACTA, vol. 73, 1990, pages 326 - 336, XP009060656, DOI: 10.1002/hlca.19900730212
- NAGASE HIROSHI ET AL.: "Synthesis of a Stable Iminium Salt and Propel lane Derivatives", THE JOURNAL OF ORGANIC CHEMISTRY, vol. 73, no. 20, 17 October 2008 (2008-10-17), pages 8093 - 8096, XP055093356
- WATANABE YOSHIKAZU ET AL.: "Design and synthesis of novel d opioid receptor agonists with an azatricyclodecane skeleton for improving blood-brain barrier penetration", BIOORGANIC & MEDICINAL CHEMISTRY LETTERS, vol. 27, 2017, pages 3495 - 3498, XP085117770, DOI: 10.1016/j.bmcl.2017.05.072
- HAYASHIDA KOHEI ET AL.: "Novel delta opioid receptor agonists with oxazatricyclodecane structure showing potent agonistic activities", BIOORGANIC & MEDICINAL CHEMISTRY LETTERS, vol. 27, no. 12, 1 June 2017 (2017-06-01), pages 2742 - 2745, XP055786181
- SAWA Y. K . ET AL.: "ELIMINATION OF THE 4-HYDROXYL GROUP OF THE ALKALOIDS RELATED TO MORPHINE-IV", TETRAHEDRON, vol. 20, no. 10, January 1964 (1964-01-01), pages 2255 - 2258, XP055786425

## Description

### Technical Field

The present invention relates to a method for producing a morphinan derivative, and a novel morphinan derivative that can be used as a starting material of the production method.

The present application claims priority based on Patent Application No. 2019-134051 filed in Japan on July 19, 2019.

### Background Art

Three types of opioid receptors, µ, δ, and κ, are known, and morphine that exhibits strong affinity for the µ receptor has been used as an analgesic drug for a long time. Though the analgesic action of morphine is strong, morphine is known to cause adverse events such as dependence formation, respiratory depression, and constipation through µ receptors.

Meanwhile, the δ receptor also has an analgesic action, and the δ receptor agonist is known not to be involved in adverse events seen in morphine.

Thus, presumably, an agonist selective for the δ receptor can become an analgesic drug superior to morphine, and research on the creation thereof is actively conducted. However, there are still no δ receptor agonists approved as therapeutic or prophylactic agents.

Patent Literature 1 and Patent Literature 2 disclose morphinan derivatives having excellent δ receptor agonist activity and represented by the following formula.

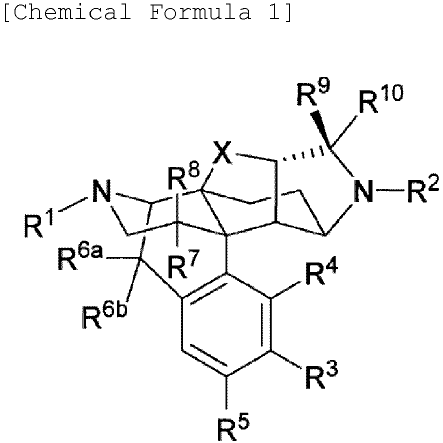

The production of these derivatives needs multi-step synthesis, and thus the yield in one reaction and the purity of the target compound obtained by each reaction are desirably high.

For example, paragraph number [0025] of Patent Literature 1 discloses a technique in which a phenyl group is introduced into a 4-position phenol form (b-1) of a morphinan derivative by Ullmann reaction to form a compound (m), and an oxygen functional group at the 4-position is removed using sodium silica gel stage I which is commercially available sodium supported on silica gel and ethylenediamine to form a compound (n).

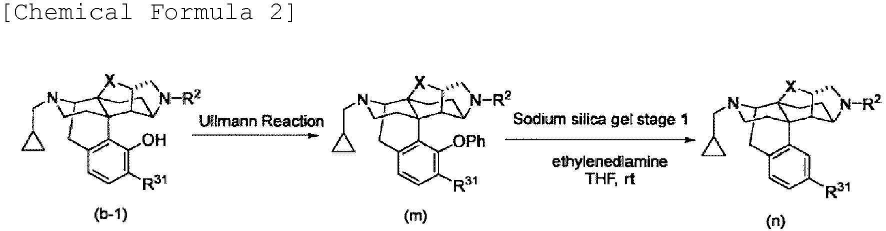

However, for example, there is a problem that the sodium supported on silica gel used in the above reaction is expensive, obtaining thereof in large quantities is difficult, and industrial use thereof is difficult.

Meanwhile, many examples in which metal sodium is used for a similar dephenoxylation reaction have been reported. For example, Non-Patent Literature 1 discloses a synthesis example of a compound 2 that is dephenoxylated by allowing metal sodium to act on a compound 1 having a 2,4-diaminophenyloxy group on a benzene ring of a morphinan skeleton in liquid ammonia.

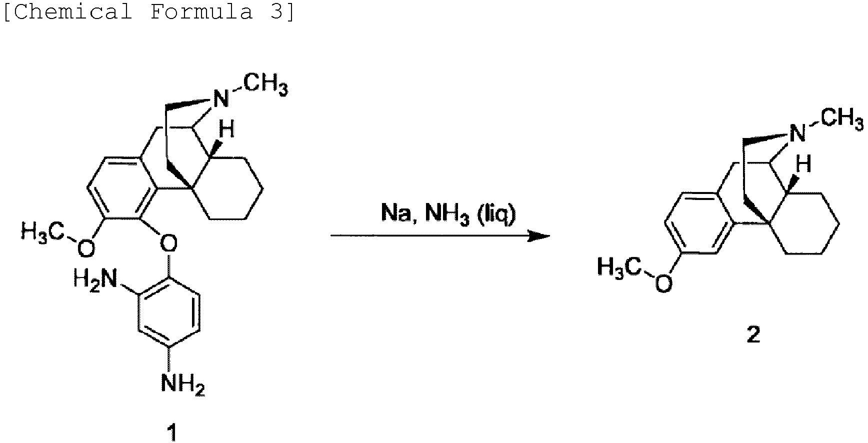

EXAMPLE 1 of Patent Literature 3 discloses an example of synthesis of 14β-hydroxy-3-methoxymorphinan (IV) by allowing metal sodium to act on 4-phenoxy-14β-hydroxy-3-methoxymorphinan (III) at -40°C in liquid ammonia.

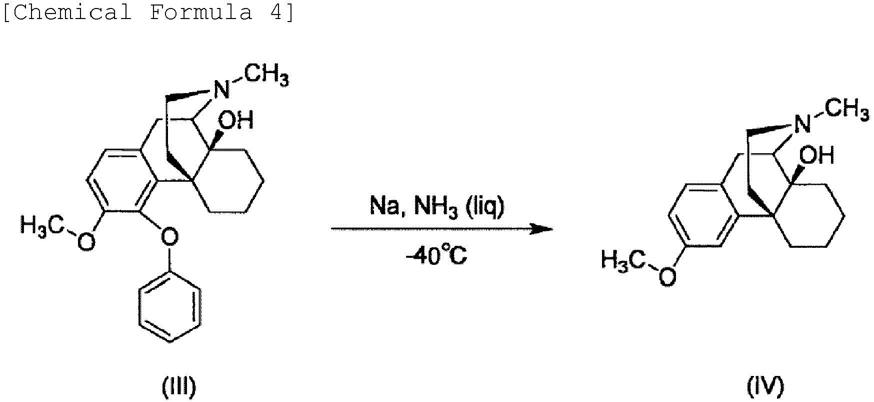

Non-Patent Literature 2 discloses a synthesis example of a compound 12 that is dephenoxylated by allowing metal sodium to act on a compound 11 having a phenoxy group at -78°C to room temperature in liquid ammonia.

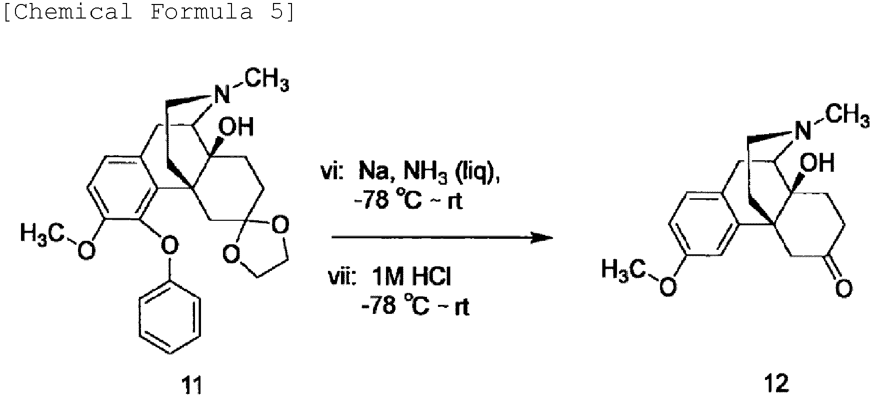

In addition, similar reactions have been also reported in Non-Patent Literatures 3 to 10.

### Citation List

### Patent Literature

Patent Literature 1: WO 2013/035833 (EP 2 774 926 A1)
Patent Literature 2: WO 2016/148232
Patent Literature 3: US 5504208

### Non-Patent Literature

Non-Patent Literature 1: Journal of Organic Chemistry, 1965, 30 (6), 1769-1773
Non-Patent Literature 2: Bioorganic & Medicinal Chemistry Letters, 19 (16), 4603-4606; 2009
Non-Patent Literature 3: Chemical Biology & Drug Design, 74 (4), 335-342; 2009
Non-Patent Literature 4: Journal of Medicinal Chemistry, 50 (11), 2747-2751; 2007
Non-Patent Literature 5: Tetrahedron, 24 (20), 6185-96; 1968
Non-Patent Literature 6: Journal of Medicinal Chemistry, 58 (12), 5038-5052; 2015
Non-Patent Literature 7: Recueil des Travaux Chimiques des Pays-Bas, 107 (6), 449-54; 1988
Non-Patent Literature 8: Helvetica Chimica Acta, 73 (2), 326-36; 1990
Non-Patent Literature 9: J. Org. Chem. 2008, 73, 8093-8096
Non-Patent Literature 10: Bioorganic & Medicinal Chemistry Letters, 27 (12), 2742-2745; 2017

### Summary of Invention

### Technical Problem

The present inventors have found that in the production of a morphinan derivative (n) from a diaryl ether derivative (m) shown in paragraph number [0025] of Patent Literature 1, the morphinan derivative (n) being a dephenoxylated form thereof, there is a problem that the cleavage position of a bond between an oxygen atom and two aryl groups cannot be controlled, and cleavage occurs at an undesired position, so that in addition to the target dephenoxylated form (n), a compound (b-1), which is a raw material, is produced as a by-product, as shown in the lower figure. The compound (b-1) produced as a by-product at this time has a structure similar to that of the target dephenoxylated form (n), and is difficult to separate from the target product (n), and purification by chromatography is required. The present inventors have found that purification by chromatography is not preferable in an industrial production method and becomes a major problem.

Also, the present inventors performed a reaction similar to that in Patent Literature 1 using metal sodium pieces applicable to an industrial production method instead of sodium supported on silica gel having a problem in application to an industrial production method in the production of the compound (n) from the compound (m), and have found a problem that the reaction is not completed and a raw material remains even when a large excess of metal sodium pieces is used relative to the compound (m).

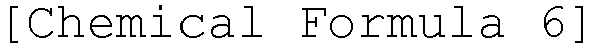

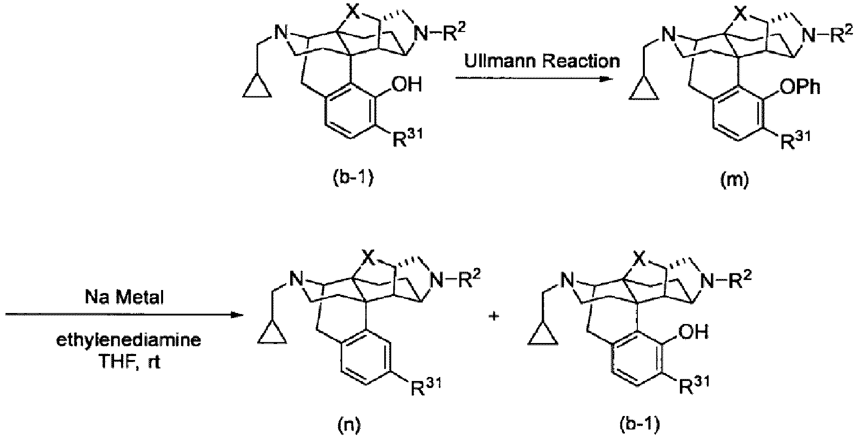

Thus, one object of the present invention is to provide a method for highly selectively producing a morphinan derivative (n) from a diaryl ether derivative (m) by suppressing production of a phenol form (b-1) as a by-product, the method being applicable to an industrial production method.

As a result of intensive studies on a production method of a morphinan derivative (n) described in paragraph number [0025] of Patent Literature 1, the present inventors have found that by using a compound in which a substituent is introduced at a specific position of a phenyl group introduced by an Ullmann reaction in the compound (b-1) as a starting material for a dearyloxylation reaction instead of the compound (m), high control of a cleavage position between oxygen and aryl groups becomes possible, and the target compound (n) is obtained with high selectivity. In addition, the present inventors have found that the target compound (n) can be obtained in a high yield by using a sodium dispersion which is also applicable to an industrial production method, thereby completing the present invention.

### Solution to Problem

That is, the present invention relates to:
[1] A method for producing a morphinan derivative represented by General Formula (II) below: 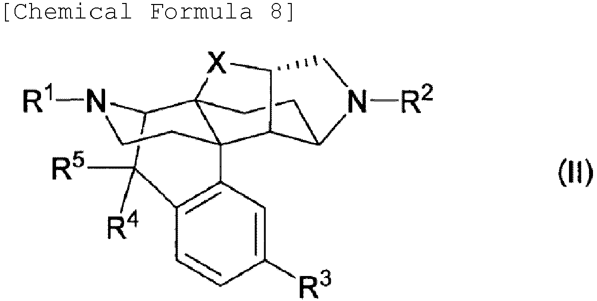
   wherein R¹ represents a hydrogen atom, a C₁₋₁₀ alkyl group optionally having a substituent, a C₃₋₆ cycloalkyl group optionally having a substituent, a C₂₋₆ alkenyl group optionally having a substituent, a C₆₋₁₀ aryl group optionally having a substituent, or a heteroaryl group optionally having a substituent,
   R² represents an amino protecting group selected from a carbamate protecting group, a sulfonamide protecting group, an acyl protecting group, and a C₇₋₁₉ aralkyl group,
   R³ represents a hydrogen atom, a halogen atom, or a C₁₋₁₀ alkoxy group optionally having a substituent,
   R⁴ and R⁵ are same or different and represent a hydrogen atom or a hydroxy group, and
   X represents O or CH₂,
   comprising the step of:
      allowing metal sodium and ethylenediamine to act on a morphinan derivative represented by
      General Formula (I) below: 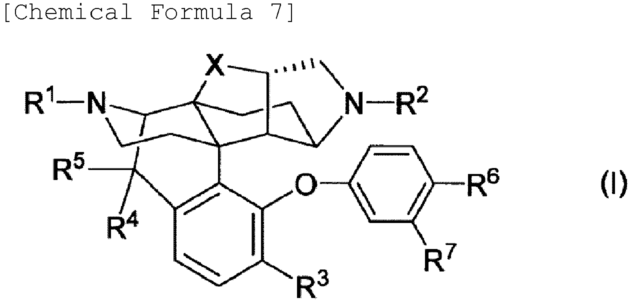
      wherein R¹ to R⁵ and X have a meaning same as above,
      R⁶ and R⁷ are same or different and represent a hydrogen atom or an electron-donating group,
      wherein R⁶ and R⁷ are not simultaneously a hydrogen atom, and
      in presence of an auxiliary solvent.
[2] The present invention also relates to the method for producing a morphinan derivative according to [1], wherein R² is a benzyl group.
[3] The present invention also relates to the method for producing a morphinan derivative according to [1] or [2], wherein R³ is a methoxy group.
[4] The present invention also relates to the method for producing a morphinan derivative according to any one of [1] to [3] above, wherein R⁶ is a C₁₋₁₀ alkyl group, OR⁸ (R⁸ represents a hydrogen atom or a C₁₋₁₀ alkyl group optionally having a substituent), or NR⁹R¹⁰ (R⁹ and R¹⁰ are same or different and represent a hydrogen atom or a C₁₋₁₀ alkyl group optionally having a substituent).
[5] The present invention also relates to the method for producing a morphinan derivative according to any one of [1] to [3] above, wherein R⁶ is a hydrogen atom.
[6] The present invention also relates to the method for producing a morphinan derivative according to any one of [1] to [4] above, wherein R⁷ is a hydrogen atom.
[7] The present invention also relates to the method for producing a morphinan derivative according to any one of [1] to [5] above, wherein R⁷ is a C₁₋₁₀ alkyl group optionally having a substituent, OR¹¹ (R¹¹ represents a hydrogen atom or a C₁₋₁₀ alkyl group optionally having a substituent), or NR¹²R¹³ (R¹² and R¹³ are same or different and represent a hydrogen atom or a C₁₋₁₀ alkyl group optionally having a substituent).
[8] The present invention also relates to the method for producing a morphinan derivative according to any one of [1] to [7] above, wherein the metal sodium is used in an amount of 1 to 10 equivalents relative to the morphinan derivative of the General Formula (I).
[9] The present invention also relates to the method for producing a morphinan derivative according to any one of [1] to [8] above, wherein the metal sodium is a metal sodium dispersion.
[10] The present invention also relates to the method for producing a morphinan derivative according to any one of [1] to [9] above, wherein the auxiliary solvent is an aromatic hydrocarbon solvent or an ether solvent.
[11] The present invention also relates to the method for producing a morphinan derivative according to any one of [1] to [10] above, wherein a reaction temperature is -30°C to 150°C.
[12] The present invention also relates to the method for producing a morphinan derivative according to any one of [1] to [11] above, wherein a reaction temperature is 0°C to 30°C.
[13] The present invention also relates to a morphinan derivative represented by General Formula (I) below: 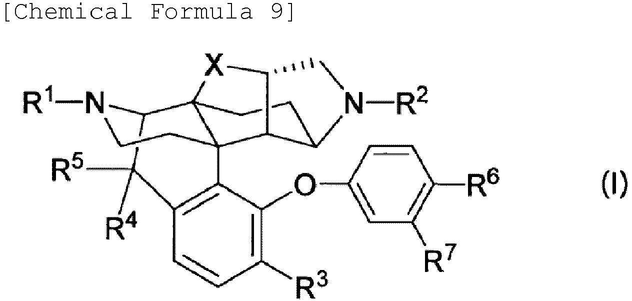
   wherein R¹ represents a hydrogen atom, a C₁₋₁₀ alkyl group optionally having a substituent, a C₃₋₆ cycloalkyl group optionally having a substituent, a C₂₋₆ alkenyl group optionally having a substituent, a C₆₋₁₀ aryl group optionally having a substituent, or a heteroaryl group optionally having a substituent,
   R² represents an amino protecting group selected from a carbamate protecting group, a sulfonamide protecting group, an acyl protecting group, and a C₇₋₁₉ aralkyl group,
   R³ represents a hydrogen atom, a halogen atom, or a C₁₋₁₀ alkoxy group optionally having a substituent,
   R⁴ and R⁵ are same or different and represent a hydrogen atom or a hydroxy group,
   R⁶ and R⁷ are same or different and represent a hydrogen atom or an electron-donating group,
   wherein R⁶ and R⁷ are not simultaneously a hydrogen atom, and
   X represents O or CH₂.
[14] The present invention, the morphinan derivative according to [13] above, wherein R² is a benzyl group.
[15] The present invention also relates to the morphinan derivative according to [13] or [14]above, wherein R³ is a methoxy group.
[16] The present invention also relates to the morphinan derivative according to any one of [13] to [15] above, wherein R⁶ is a C₁₋₁₀ alkyl group, OR⁸ (R⁸ represents a hydrogen atom or a C₁₋₁₀ alkyl group optionally having a substituent), or NR⁹R¹⁰ (R⁹ and R¹⁰ are same or different and represent a hydrogen atom or a C₁₋₁₀ alkyl group optionally having a substituent).
[17] The present invention also relates to the morphinan derivative according to any one of [13] to [15] above, wherein R⁶ is a hydrogen atom.
[18] The present invention also relates to the morphinan derivative according to any one of [13] to [15] above, wherein R⁷ is a hydrogen atom.
[19] The present invention also relates to the morphinan derivative according to any one of [13] to [17] above, wherein R⁷ is a C₁₋₁₀ alkyl group optionally having a substituent, OR¹¹ (R¹¹ represents a hydrogen atom or a C₁₋₁₀ alkyl group optionally having a substituent), or NR¹²R¹³ (R¹² and R¹³ are same or different and represent a hydrogen atom or a C₁₋₁₀ alkyl group optionally having a substituent).
[20] Further, the present invention relates to a morphinan derivative according to [13], wherein the derivative is selected from
   (1S,3aR,5aS,6R,11bS,11cS)-3-benzyl-11-(4-(tert-butyl)phenoxy)-14-(cyclopropylmethyl)-10-methoxy-2,3,3a,4,5,6,7,11c-octahydro-1H-6,11b-(epiminoethano)-1,5a-methanonaphtho[1,2-e]indole,
   (1S,3aR,5aS,6R,11bS,11cS)-3-benzyl-14-(cyclopropylmethyl)-10-methoxy-11-(3-methoxyphenoxy)-2,3,3a,4,5,6,7,11c-octahydro-1H-6,11b-(epiminoethano)-1,5a-methanonaphtho[1,2-e]indole, and
   (1S,3aR,5aS,6R,11bS,11cS)-3-benzyl-14-(cyclopropylmethyl)-10-methoxy-11-(p-toluyloxy)-2,3,3a,4,5,6,7,11c-octahydro-1H-6,11b-(epiminoethano)-1,5a-methanonaphtho[1,2-e]indole.

### Advantageous Effects of Invention

According to the present invention, a method for highly selectively producing a morphinan derivative (n) from a diaryl ether derivative (m) by suppressing production of a phenol form (b-1) as a by-product, the method being applicable to an industrial production method can be provided.

### Description of Embodiments

The present invention will be next described in more detail.

In the General Formula (I), examples of the C₁₋₁₀ alkyl group optionally having a substituent of R¹ include a C₁₋₁₀ alkyl group optionally having a substituent selected from a halogen atom, a hydroxy group, a C₁₋₁₀ alkoxy group, an amino group and the like; a C₁₋₁₀ alkyl group substituted with a C₃₋₆ cycloalkyl optionally having a substituent; an aralkyl group optionally having a substituent; and a heteroarylalkyl group optionally having a substituent.

Examples of the C₁₋₁₀ alkyl group of the C₁₋₁₀ alkyl group optionally having a substituent of R¹ include linear or branched C₁₋₁₀ alkyl groups such as a methyl group, an ethyl group, a propyl group, an isopropyl group, a butyl group, an iso-butyl group, a tert-butyl group, a pentyl group, and a hexyl group, preferably include a methyl group, an ethyl group, and a propyl group, and more preferably include a methyl group.

Examples of the C₁₋₁₀ alkyl group substituted with a C₃₋₆ cycloalkyl optionally having a substituent of R¹ include a methyl group and an ethyl group substituted with a C₃₋₆ cycloalkyl group such as a cyclopropyl group, a cyclobutyl group, a cyclopentyl group, and a cyclohexyl group, the cycloalkyl group optionally having a substituent selected from a halogen atom, a hydroxy group, a C₁₋₁₀ alkoxy group, an amino group and the like, preferably include a cyclopropylmethyl group, a cyclopropylethyl group, a cyclobutylmethyl group, and a cyclobutylethyl group, and more preferably include a cyclopropylmethyl group.

Examples of the aralkyl group optionally having a substituent of R¹ include an aralkyl group optionally having a substituent selected from a halogen atom, a hydroxy group, a C₁₋₁₀ alkoxy group, an amino group and the like, and having a carbon number of the aryl moiety of C₆₋₁₀ and a carbon number of the alkylene moiety of C_{1_5}, preferably include a methyl group and an ethyl group substituted with phenyl or naphthyl, and still more preferably include a methyl group substituted with phenyl (that is, a benzyl group).

Examples of the heteroarylalkyl group optionally having a substituent of R¹ include a heteroarylalkyl group in which the heteroaryl moiety optionally having a substituent selected from a halogen atom, a hydroxy group, a C₁₋₁₀ alkoxy group, an amino group and the like is a heteroaryl containing 1 to 4 heteroatoms that are same or different and selected from a nitrogen atom, an oxygen atom, and a sulfur atom, as ring-constituting atoms, and the alkyl moiety is a C₁₋₆ alkyl group such as a methyl group, an ethyl group, and a propyl group, and preferably include monocyclic heteroarylalkyl groups such as a (pyridine-2-yl)methyl group, a (pyridine-3-yl)methyl group, a (pyridine-4-yl)methyl group, a 2-(pyridine-2-yl)ethyl group, a (furan-2-yl)methyl group, a (furan-3-yl)methyl group, a (imidazole-2-yl)methyl group, a (imidazole-4-yl)methyl group, a (imidazole-5-yl)methyl group, a (thiazole-2-yl)methyl group, a (thiazole-4-yl)methyl group, a (thiazole-5-yl)methyl group, a (thiophene-2-yl)methyl group, and a 2-(thiophene-2-yl)ethyl group, and bicyclic heteroarylalkyl groups such as a (quinoline-3-yl)methyl group and a (indole-3-yl)methyl group.

Examples of the C₃₋₆ cycloalkyl group optionally having a substituent of R¹ in the General Formula (I) include a cyclopropyl group, a cyclobutyl group, a cyclopentyl group, and a cyclohexyl group optionally having a substituent selected from a halogen atom, a hydroxy group, a C₁₋₁₀ alkoxy group, an amino group and the like, and preferably include a cyclopropyl group.

Examples of the C₂₋₆ alkenyl group optionally having a substituent of R¹ in the General Formula (I) include alkenyl groups optionally having a substituent selected from a halogen atom, a hydroxy group, a C₁₋₁₀ alkoxy group, an amino group and the like, such as an allyl group, a vinyl group, a 1-propenyl group, a 2-butenyl group, a 3-butenyl group, a 2-pentenyl group, a 3-pentenyl group, a 4-pentenyl group, a 2-hexenyl group, a 3-hexenyl group, a 4-hexenyl group, and a 5-hexenyl group.

Examples of the C₆₋₁₀ aryl group optionally having a substituent of R¹ in the General Formula (I) include a phenyl group and a naphthyl group optionally having a substituent selected from a halogen atom, a hydroxy group, a C₁₋₁₀ alkoxy group, an amino group and the like, and a phenyl group is preferable.

Examples of the heteroaryl group optionally having a substituent of R¹ in the General Formula (I) include a heteroaryl group optionally having a substituent selected from a halogen atom, a hydroxy group, a C₁₋₁₀ alkoxy group, an amino group and the like and containing 1 to 4 heteroatoms that are same or different and selected from a nitrogen atom, an oxygen atom, and a sulfur atom, as ring-constituting atoms, and preferably include monocyclic heteroaryl groups such as a pyridyl group, a furyl group, an imidazolyl group, a pyrimidinyl group, a pyrazinyl group, and a thiazolyl group; and bicyclic heteroaryl groups such as a quinolyl group and an indolyl group.

As mentioned above, according to the present invention R² represents an amino protecting group selected from a carbamate protecting group, a sulfonamide protecting group, an acyl protecting group, and a C₇₋₁₉ aralkyl group. Examples of the amino protecting group represented by R² include carbamate protecting groups such as a methoxycarbonyl group, an ethoxycarbonyl group, a tertbutoxycarbonyl group, a tert-amyloxycarbonyl group, a 2,2,2-trichloroethoxycarbonyl group, a benzyloxycarbonyl group, a p-chlorobenzyloxycarbonyl group, a p-methoxybenzylcarbonyl group, a p-nitrobenzyloxycarbonyl group, a p-phenylazobenzyloxycarbonyl group, a p-methoxyphenylazobenzyloxycarbonyl group, a 3,5-dimethoxybenzyloxycarbonyl group, a 3,4,5-trimethoxybenzyloxycarbonyl group, a p-biphenylisopropyloxycarbonyl group, a diisopropylmethyloxycarbonyl group, a 2-(trimethylsilyl)ethoxycarbonyl group, and a 9-fluorenylmethyloxycarbonyl group; sulfonamide protecting groups such as a p-toluenesulfonyl group and a 2-nitrobenzenesulfonyl group; acyl protecting groups such as an acetyl group and a trifluoroacetyl group; and C₇₋₁₉ aralkyl groups such as a benzyl group, a trityl group, and a naphthylmethyl group, preferably include a C₇₋₁₉ aralkyl group, and more preferably include a benzyl group.

Examples of the C₁₋₁₀ alkoxy group optionally having a substituent of R³ include linear or branched C₁₋₁₀ alkoxy groups optionally having a substituent selected from a halogen atom, a hydroxy group, a C₁₋₁₀ alkoxy group, an amino group and the like, such as a methoxy group, an ethoxy group, a propoxy group, an iso-propoxy group, a butoxy group, an iso-butoxy group, a tert-butoxy group, a pentyloxy group, and a hexyloxy group, preferably include a methoxy group, an ethoxy group, and a propoxy group, and more preferably include a methoxy group.

Examples of the electron-donating group represented by R⁶ include a C₁₋₁₀ alkyl group optionally having a substituent selected from a halogen atom, a hydroxy group, a C₁₋₁₀ alkoxy group, an amino group and the like, OR⁸ (R⁸ represents a hydrogen atom or a C₁₋₁₀ alkyl group optionally having a substituent), and NR⁹R¹⁰ wherein R⁹ and R¹⁰ are same or different and represent a hydrogen atom or a C₁₋₁₀ alkyl group optionally having a substituent.

Examples of the C₁₋₁₀ alkyl group optionally having a substituent of R⁸ include linear or branched C₁₋₁₀ alkyl groups such as a methyl group, an ethyl group, a propyl group, an isopropyl group, a butyl group, an iso-butyl group, a tert-butyl group, a pentyl group, and a hexyl group, and preferably include a tert-butyl group.

Examples of R⁹ and R¹⁰ include an amino group, a methylamino group, a dimethylamino group, an ethylamino group, a diethylamino group, and a propylamino group.

R⁶ is preferably a C₁₋₁₀ alkyl group, and examples thereof more preferably include a tert-butyl group.

Examples of the electron-donating group represented by R⁷ include a C₁₋₁₀ alkyl group optionally having a substituent selected from a halogen atom, a hydroxy group, a C₁₋₁₀ alkoxy group, an amino group and the like, OR¹¹ (R¹¹ represents a hydrogen atom or a C₁₋₁₀ alkyl group optionally having a substituent), and NR¹²R¹³ (R¹² and R¹³ are same or different and represent a hydrogen atom or a C₁₋₁₀ alkyl group optionally having a substituent).

Examples of the C₁₋₁₀ alkyl group optionally having a substituent of R¹¹ include the same as those of the C₁₋₁₀ alkyl group optionally having a substituent of R⁸, and examples of R¹² and R¹³ include the same as those of R⁹ and R¹⁰.

Examples of X include an oxygen atom and CH₂, and CH₂ is preferable.

In the present specification, examples of the halogen atom include a fluorine atom, a chlorine atom, a bromine atom, and an iodine atom, preferably include a fluorine atom and a chlorine atom, and more preferably include a fluorine atom.

In the present specification, examples of the C₁₋₁₀ alkoxy group include linear or branched C₁₋₁₀ alkoxy groups such as a methoxy group, an ethoxy group, a propoxy group, an iso-propoxy group, a butoxy group, an iso-butoxy group, a tert-butoxy group, a pentyloxy group, and a hexyloxy group, preferably include a methoxy group, an ethoxy group, and a propoxy group, and more preferably include a methoxy group.

In the present specification, examples of the amino group include NR^{d}R^{e} wherein R^{d} and R^{e} are same or different and examples thereof include a hydrogen atom and a C₁₋₁₀ alkyl group optionally having a substituent, and examples of the amino group preferably include an amino group, a dimethylamino group, and a diethylamino group.

Preferable combinations of these substituents include a case where
R¹ is a C₁₋₁₀ alkyl group optionally having a substituent or a C₁₋₁₀ alkyl group substituted with a C₃₋₆ cycloalkyl optionally having a substituent;
R² is an amino protecting group selected from a carbamate protecting group, a sulfonamide protecting group, an acyl protecting group, and a C₇₋₁₉ aralkyl group;
R³ is a methoxy group;
R⁴ and R⁵ are a hydrogen atom;
R⁶ is a hydrogen atom or a C₁₋₁₀ alkyl group;
R⁷ is a hydrogen atom or a methoxy group;
provided that one of R⁶ and R⁷ is a hydrogen atom, and the other is other than a hydrogen atom; and
X is CH₂.

The compound represented by the General Formula (I) can be produced by a known method, for example, the method described in Patent Literature 1.

The production of the morphinan derivative represented by General Formula (II) can be performed by allowing metal sodium to act on the compound of the General Formula (I) in presence of ethylenediamine.

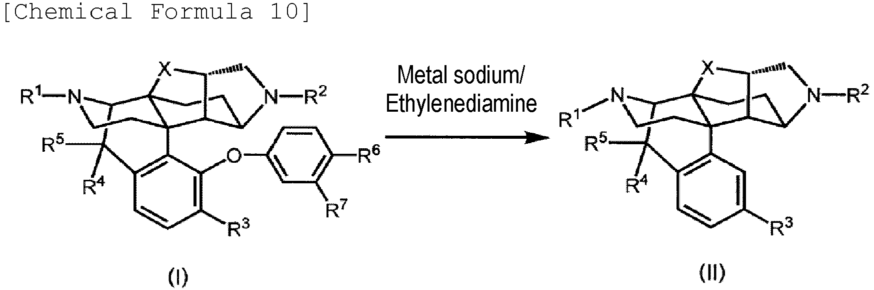

As the sodium reagent used in the above reaction, commercially available sodium reagents such as metal sodium and a metal sodium dispersion can be used, and a metal sodium dispersion is preferable.

In the present specification, the metal sodium dispersion refers to one obtained by dissolving sodium in an inert solvent having a boiling point of 100°C or higher, and gradually cooling the solution while strongly stirring it to solidify the sodium. Examples of the particle size of the metal sodium dispersion used include 1 to 100 µm, preferably include 1 to 75 µm, more preferably include 1 to 50 µm, and still more preferably include 1 to 25 µm.

The amount of the sodium reagent used in the reaction is 1 to 50 equivalents, preferably 1 to 30 equivalents, more preferably 1 to 20 equivalents, still more preferably 1 to 10 equivalents, preferably 2 to 10 equivalents, and preferably 5 to 10 equivalents relative to the raw material.

Though other primary amines (for example, ethylamine, propylamine and the like) can be used instead of the ethylenediamine used in the above reaction, ethylenediamine is preferably used.

The amount of ethylenediamine or other primary amines used can be 1 to 100 mol, preferably 1 to 50 mol, more preferably 1 to 30 mol, still more preferably 1 to 20 mol, and more preferably 1.5 to 20 mol, relative to 1 mol of the sodium reagent.

Examples of the auxiliary solvent used in the above reaction include an aromatic hydrocarbon solvent and an ether solvent, examples of the aromatic hydrocarbon solvent include benzene, toluene, and xylene, and preferably include toluene, and examples of the ether solvent include tetrahydrofuran and 1,4-dioxane, and preferably include tetrahydrofuran.

Among these solvents, preferable examples include tetrahydrofuran.

For the amount of the auxiliary solvent used, though the amount of an organic solvent used is not particularly limited, it is preferably in a range of 1 to 50 parts by mass, and preferably in a range of 1 to 20 parts by mass relative to 1 part by mass of the morphinan derivative represented by the General Formula (I). These organic solvents are preferably used after being purified by a known method such as distillation.

The above reaction is preferably performed under an inert gas such as nitrogen or argon, the reaction temperature can be in the range of -30 to 150°C, preferably in the range of -10 to 120°C, and more preferably in the range of -5 to 115°C, and the target product can be obtained by reaction of 1 to 16 hours.

The morphinan derivative represented by General Formula (II) produced by the above method can be isolated and/or purified by applying a method known per se, for example, solvent extraction, concentration, crystallization, precipitation, filtration, chromatography, or the like. When the production method of the present invention is applied as an industrial production method, chromatography is preferably not used as a purification method from the viewpoint of production efficiency.

Thus, one embodiment of the present invention is the production method, including at least one step selected from solvent extraction, concentration, crystallization, precipitation, and filtration as the isolation and/or purification step of the morphinan derivative represented by General Formula (II), and not including the step of chromatography.

By applying the isolation and/or purification step, the morphinan derivative represented by General Formula (II) can be obtained, for example, with a purity of 75% or more, preferably with a purity of 80% or more, more preferably with a purity of 90% or more, still more preferably with a purity of 94% or more, and with a yield of 75% or more, preferably with a yield of 80% or more, more preferably with a yield of 85% or more, still more preferably with a yield of 90% or more, particularly preferably with a yield of 95% or more. At this time, the content of the phenol form (b-1) as a by-product in the target product can be 5% by mass or less, preferably 3% by mass or less, more preferably 1% by mass or less, and still more preferably 0% by mass (that is, the phenol form (b-1) is not contained), relative to the mass of the target product.

### Examples

Hereinafter, the present invention will be described in more detail with reference to Examples, Reference Examples, Comparative Examples, and Test Examples, but the present invention is not limited thereto. For the naming of the compounds of Examples, the compounds of Reference Examples, and the compounds of Comparative Examples, the structural formulas drawn using ChemDraw ver. 15 manufactured by CambridgeSoft were converted into English names by a naming algorithm equipped in the same software, and then translated into Japanese.

### 1. Production of starting material: diaryl ether derivative

A production example of a diphenyl ether derivative by Ullmann coupling of bromobenzene with (1S,3aR,5aS,6R,11bS,11cS)-3-benzyl-14-(cyclopropylmethyl)-10-methoxy-2,3,3a,4,5,6,7,11c-octahydro-1H-6,11b-(epiminoethano)-1,5a-methanonaphtho[1,2-e]indole-11-ol will be shown.

### (Reference Example 1)

### Synthesis of (1S,3aR,5aS,6R,11bS,11cS)-3-benzyl-14-(cyclopropylmethyl)-10-methoxy-11-phenoxy-2,3,3a,4,5,6,7,11c-octahydro-1H-6,11b-(epiminoethano)-1,5a-methanonaphtho[1,2-e]indole

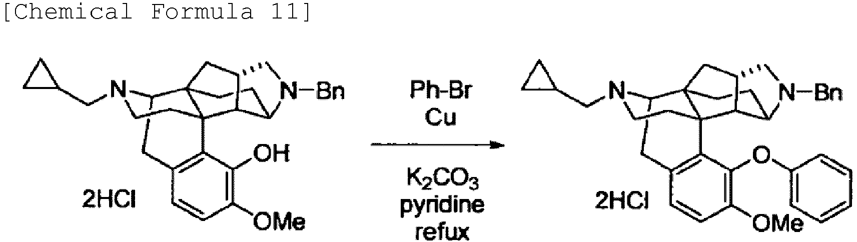

In a 300 mL round bottom flask, under a nitrogen atmosphere, (1S,3aR,5aS,6R,11bS,11cS)-3-benzyl-14-(cyclopropylmethyl)-10-methoxy-2,3,3a,4,5,6,7,11c-octahydro-1H-6,11b-(epiminoethano)-1,5a-methanonaphtho[1,2-e]indole-11-ol dihydrochloride (5 g, 1 equivalent) was dissolved in pyridine (50 mL). Potassium carbonate (3.8 g, 6 equivalents), bromobenzene (4.8 mL, 5 equivalents), and copper powder (0.6 g, 1 equivalent) were added to the obtained solution, and the mixture was heated and refluxed while being vigorously stirred. After 16 hours, the reaction was analyzed by mass spectrometry (MS) and thin layer chromatography, and the disappearance of the raw material was confirmed.

After cooling the reaction mixture to room temperature, the reaction mixture was filtered through a celite pad to remove insolubles. The pad was washed with ethyl acetate (50 mL) and methanol (50 mL), and 6% aqueous ammonia (50 mL) was added to the combined filtrate and washings. The organic layer and the aqueous layer were separated with a separating funnel, and the organic layer was fractionated. Further, the aqueous layer was extracted with ethyl acetate (50 mL × 2 times). The combined organic layers were dried over anhydrous sodium sulfate, the anhydrous sodium sulfate was filtered off, and then the combined organic layers were concentrated under reduced pressure to obtain a title compound crude product.

The obtained crude product was dissolved in a 1 : 1 mixture (100 mL) of n-heptane and ethyl acetate, the solution was filtered through a short column packed with silica gel (35 g) using the same mixed solvent as an elution solvent, and the obtained filtrate was concentrated under reduced pressure to obtain the title compound (free form).

The obtained free form was dissolved in a 1 : 4 mixed liquid (50 mL) of n-heptane and ethyl acetate, and then a 4 N hydrogen chloride/ethyl acetate solution (5 mL) was slowly added at 0 to 5°C with stirring to obtain a suspension.

The resulting solid was collected by filtration to obtain a hydrochloride of the title compound (5 g).

The obtained hydrochloride of the title compound was desalted with a 4 N aqueous sodium hydroxide solution (20 mL) to obtain the title compound (4.4 g, yield: 88%).

¹H-NMR (400 MHz, CDCl₃): δ7.10 - 7.35 (m, 7H), 6.90 - 7.05 (m, 2H), 6.70 - 6.85 (m, 3H), 6.72 (d, 1H, J = 13 Hz), 3.68 (s, 3H), 3.58 (d, 1H, J = 13 Hz), 2.90 - 3.25 (m, 6H), 2.60 - 2.75 (m, 3H), 2.45 (dd, 1H, J = 5, 12 Hz), 2.29 (d, 2H, J = 6 Hz), 1.99 (dt, 1H, J = 3, 13 Hz), 1.50 - 1.80 (m, 3H), 1.10 - 1.25 (m, 1H), 1.00 - 1.10 (m, 2H), 0.70 - 0.85 (m, 2H), 0.40 - 0.55 (m, 2H), 0.00 - 0.15 (m, 2H).

A production example of a diaryl ether derivative by Ullmann coupling of other haloaryls with (1S,3aR,5aS,6R,11bS,11cS)-3-benzyl-14-(cyclopropylmethyl)-10-methoxy-2,3,3a,4,5,6,7,11c-octahydro-1H-6,11b-(epiminoethano)-1,5a-methanonaphtho[1,2-e]indole-11-ol will be shown below.

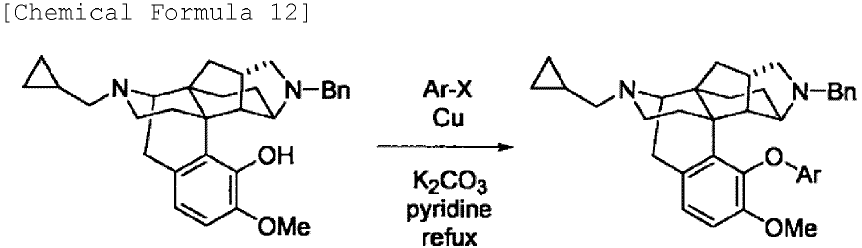

### (Example 1)

### Synthesis of (1S,3aR,5aS,6R,11bS,11cS)-3-benzyl-11-(4-(tert-butyl)phenoxy)-14-(cyclopropylmethyl)-10-methoxy-2,3,3a,4,5,6,7,11c-octahydro-1H-6,11b-(epiminoethano)-1,5a-methanonaphtho[1,2-e]indole

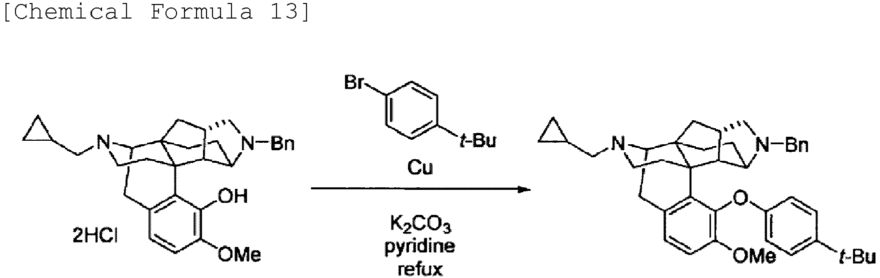

In a 2000 mL round bottom flask, under a nitrogen atmosphere, (1S,3aR,5aS,6R,11bS,11cS)-3-benzyl-14-(cyclopropylmethyl)-10-methoxy-2,3,3a,4,5,6,7,11c-octahydro-1H-6,11b-(epiminoethano)-1,5a-methanonaphtho[1,2-e]indole-11-ol dihydrochloride (30 g, 1 equivalent) was dissolved in pyridine (300 mL). Potassium carbonate (22.9 g, 3 equivalents), 4-tert-butylbromobenzene (47 mL, 5 equivalents), and copper powder (3.5 g, 1 equivalent) were added to the obtained solution, and the mixture was heated and refluxed while being vigorously stirred. After 16 hours, the reaction was analyzed by MS and thin layer chromatography to confirm that a small amount of the raw material remains. The temperature of the reaction liquid was once returned to room temperature, potassium carbonate (22.9 g, 3 equivalents), 4-tert-butylbromobenzene (47 mL, 5 equivalents), copper powder (3.5 g, 1 equivalent), and pyridine (300 mL) were added to the reaction liquid, and the mixture was heated and refluxed again while being vigorously stirred. After 24 hours, the reaction was analyzed by MS and thin layer chromatography, and complete disappearance of the raw material was confirmed.

After cooling the reaction mixture to room temperature, the reaction mixture was filtered through a celite pad to remove insolubles. The pad was washed with ethyl acetate (300 mL) and methanol (100 mL), and 6% aqueous ammonia (300 mL) was added to the combined filtrate and washings. The organic layer and the aqueous layer were separated with a separating funnel, and the aqueous layer was extracted with ethyl acetate (300 mL × 2 times). The combined organic layers were dried over anhydrous sodium sulfate, the anhydrous sodium sulfate was filtered off, and then the combined organic layers were concentrated under reduced pressure to obtain a title compound crude product.

The obtained crude product was dissolved in a 1 : 1 mixture (300 mL) of n-heptane and ethyl acetate, the solution was filtered through a short column packed with silica gel (210 g) using the same mixed solvent as an elution solvent (2100 mL), and the obtained filtrate was concentrated under reduced pressure to obtain the title compound (free form). The obtained free form was dissolved in a 1 : 4 mixed liquid (150 mL) of n-heptane and ethyl acetate, and then a 4 N hydrogen chloride/ethyl acetate solution (30 mL) was slowly added at 0 to 5°C with stirring to obtain a suspension.

The resulting solid was collected by filtration to obtain the title compound (33 g) as a hydrochloride.

The obtained hydrochloride of the title compound was desalted with a 4 N aqueous sodium hydroxide solution (20 mL) to obtain the title compound (29.2 g, yield: 88%).

¹H-NMR (400 MHz, CDCl₃): δ7.10 - 7.35 (m, 7H), 6.97 (d, 1H, J = 8 Hz), 6.77 (d, 1H, J = 8 Hz), 6.67 (d, 2H, J = 8 Hz), 3.70 (d, 1H, J = 13 Hz), 3.68 (s, 3H), 3.55 (d, 1H, J = 13 Hz), 2.90 - 3.20 (m, 6H), 2.60 - 2.70 (m, 1H), 2.46 (dd, 1H, J = 4, 12 Hz), 2.29 (d, 2H, J = 6 Hz), 1.99 (dt, 1H, J = 3, 13 Hz), 1.75 (dt, 1H, J = 5, 13 Hz), 1.50 - 1.65 (m, 3H), 1.30 (s, 9H), 1.20 - 1.35 (m, 1H), 1.10 - 1.20 (m, 1H), 1.00 - 1.10 (m, 2H), 0.70 - 0.85 (m, 2H), 0.40 - 0.50 (m, 2H), 0.00 - 0.15 (m, 2H).

### (Example 2)

### Synthesis of (1S,3aR,5aS,6R,11bS,11cS)-3-benzyl-14-(cyclopropylmethyl)-10-methoxy-11-(3-methoxyphenoxy)-2,3,3a,4,5,6,7,11c-octahydro-1H-6,11b-(epiminoethano)-1,5a-methanonaphtho[1,2-e]indole

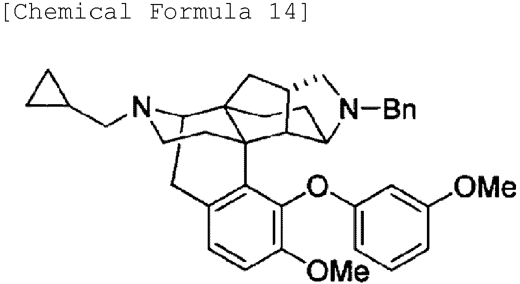

By a similar method to that in Example 1, using 3-bromoanisole as a halobenzene, the title compound (0.12 g, yield: 99%) was obtained.

¹H-NMR (400 MHz, CDCl₃): δ7.15 - 7.30 (m, 5H), 7.11 (t, 1H, J = 8 Hz), 6.97 (d, 1H, J = 8 Hz), 6.78 (d, 1H, J = 8 Hz), 6.52 (dd, 1H, J = 2, 8 Hz), 6.38 (t, 1H, J = 2 Hz), 6.32 (dd, 1H, J = 2, 8 Hz), 3.75 (s, 3H), 3.72 (d, 1H, J = 13 Hz), 3.68 (s, 3H), 3.58 (d, 1H, J = 13 Hz), 2.90 - 3.25 (m, 6H), 2.60 - 2.75 (m, 3H), 2.47 (dd, 1H, J = 4, 12 Hz), 2.29 (d, 2H, J = 6 Hz), 1.99 (dt, 1H, J = 3, 13 Hz), 1.76 (dt, 1H, J = 5, 13 Hz), 1.50 - 1.70 (m, 2H), 1.10 - 1.20 (m, 1H), 1.00 - 1.10 (m, 2H), 0.70 - 0.85 (m, 2H), 0.40 - 0.50 (m, 2H), 0.00 - 0.15 (m, 2H).

### (Example 3)

### Synthesis of (1S,3aR,5aS,6R,11bS,11cS)-3-benzyl-14-(cyclopropylmethyl)-10-methoxy-11-(p-toluyloxy)-2,3,3a,4,5,6,7,11c-octahydro-1H-6,11b-(epiminoethano)-1,5a-methanonaphtho[1,2-e]indole

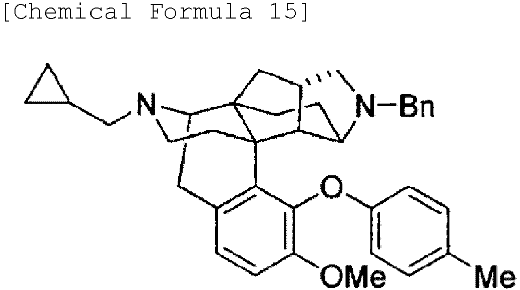

By a similar method to that in Example 1, using 4-bromotoluene as a halobenzene, the title compound (0.11 g, yield: 90%) was obtained.

¹H-NMR (400 MHz, CDCl₃): δ7.15 - 7.30 (m, 5H), 7.02 (d, 2H, J = 8 Hz), 6.97 (d, 1H, J = 8 Hz), 6.77 (d, 1H, J = 8 Hz), 6.65 (d, 2H, J = 8 Hz), 3.73 (d, 1H, J = 13 Hz), 3.67 (s, 3H), 3.59 (d, 1H, J = 13 Hz), 2.90 - 3.25 (m, 6H), 2.60 - 2.75 (m, 3H), 2.47 (dd, 1H, J = 4, 12 Hz), 2.35 (d, 2H, J = 6 Hz), 2.29 (s, 3H), 1.99 (dt, 1H, J = 3, 13 Hz), 1.76 (dt, 1H, J = 5, 13 Hz), 1.50 - 1.70 (m, 2H), 1.10 - 1.20 (m, 1H), 1.00 - 1.10 (m, 2H), 0.70 - 0.85 (m, 2H), 0.40 - 0.50 (m, 2H), 0.00 - 0.15 (m, 2H).

### (Comparative Example 1)

### Synthesis of ethyl 3-(((1S,3aR,5aS, 6R,11bS,11cS)-3-benzyl-14-(cyclopropylmethyl)-10-methoxy-2,3,3a,4,5,6,7,11c-octahydro-1H-6,11b-(epiminoethano)-1,5a-methanonaphtho[1,2-e]indole-11-yl)oxy)benzoate

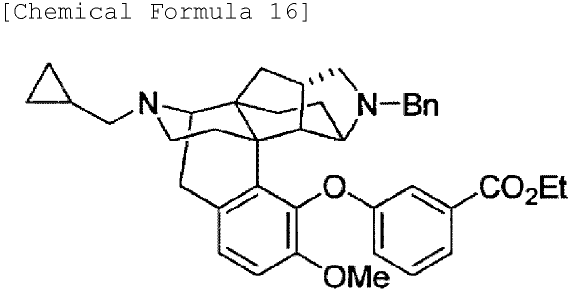

By a similar method to that in Example 1, using ethyl 3-iodobenzoate as a halobenzene, the title compound (0.19 g, yield: 73%) was obtained.

¹H-NMR (400 MHz, CDCl₃): δ7.65 (d, 1H, J = 8 Hz), 7.48 (s, 1H), 7.15 - 7.35 (m, 6H), 7.00 (d, 1H, J = 8 Hz), 6.91 (dd, 1H, J = 3, 8 Hz), 6.78 (d, 1H, J = 8 Hz), 4.46 (q, 2H, J = 7 Hz), 3.71 (d, 1H, J = 13 Hz), 3.65 (s, 3H), 3.57 (d, 1H, J = 13 Hz), 2.90 - 3.20 (m, 6H), 2.60 - 2.80 (m, 2H), 2.46 (dd, 1H, J = 4, 12 Hz), 2.29 (d, 2H, J = 6 Hz), 2.00 (dt, 1H, J = 3, 13 Hz), 1.76 (dt, 1H, J = 5, 13 Hz), 1.50 - 1.70 (m, 3H), 1.38 (t, 3H, J = 7 Hz), 1.10 - 1.25 (m, 1H), 1.00 - 1.10 (m, 2H), 0.70 - 0.85 (m, 2H), 0.40 - 0.50 (m, 2H), 0.00 - 0.15 (m, 2H).

### (Comparative Example 2)

### Synthesis of 3-(((1S,3aR,5aS,6R,11bS,11cS)-3-benzyl-14-(cyclopropylmethyl)-10-methoxy-2,3,3a,4,5,6,7,11c-octahydro-1H-6,11b-(epiminoethano)-1,5a-methanonaphtho[1,2-e]indole-11-yl)oxy)benzonitrile

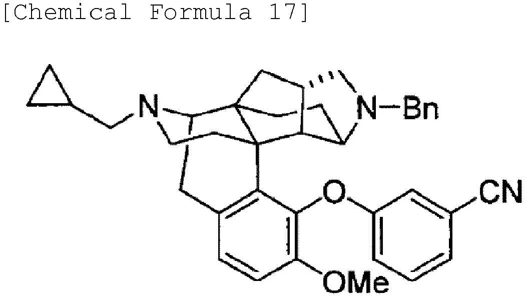

By a similar method to that in Example 1, using 3-bromobenzonitrile as a halobenzene, the title compound (0.05 g, yield: 44%) was obtained.

¹H-NMR (400 MHz, CDCl₃): δ7.15 - 7.40 (m, 7H), 6.90 - 7.05 (m, 3H), 6.78 (d, 1H, J = 8 Hz), 3.71 (d, 1H, J = 13 Hz), 3.67 (s, 3H), 3.58 (d, 1H, J = 13 Hz), 2.95 - 3.15 (m, 6H), 2.65 - 2.80 (m, 2H), 2.48 (dd, 1H, J = 4, 12 Hz), 2.25 - 2.35 (m, 2H), 2.00 (dt, 1H, J = 3, 13 Hz), 1.76 (dt, 1H, J = 5, 13 Hz), 1.50 - 1.70 (m, 3H), 1.15 - 1.30 (m, 1H), 1.00 - 1.05 (m, 1H), 0.85 - 0.95 (m, 1H), 0.65 - 0.85 (m, 2H), 0.40 - 0.55 (m, 2H), 0.00 - 0.15 (m, 2H).

### (Comparative Example 3)

### Synthesis of 4-(((1S,3aR,5aS,6R,11bS,11cS)-3-benzyl-14-(cyclopropylmethyl)-10-methoxy-2,3,3a,4,5,6,7,11c-octahydro-1H-6,11b-(epiminoethano)-1,5a-methanonaphtho[1,2-e]indole-11-yl)oxy)benzonitrile

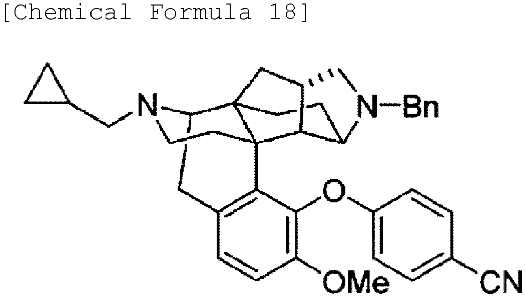

By a similar method to that in Example 1, using 4-bromobenzonitrile as a halobenzene, the title compound (0.085 g, yield: 75%) was obtained.

¹H-NMR (400 MHz, CDCl₃): δ7.52 (d, 2H, J = 8 Hz), 7.15 - 7.30 (m, 5H), 7.02 (d, 1H, J = 8 Hz), 6.81 (d, 2H, J = 8 Hz), 6.77 (d, 1H, J = 8 Hz), 3.70 (d, 1H, J = 13 Hz), 3.66 (s, 3H), 3.56 (d, 1H, J = 13 Hz), 2.90 - 3.15 (m, 6H), 2.70 - 2.80 (m, 1H), 2.60 - 2.70 (m, 1H), 2.47 (dd, 1H, J = 4, 12 Hz), 2.20 - 2.35 (m, 2H), 1.95 (dt, 1H, J = 3, 13 Hz), 1.76 (dt, 1H, J = 5, 13 Hz), 1.50 - 1.70 (m, 3H), 1.15 - 1.30 (m, 1H), 1.00 - 1.10 (m, 1H), 0.90 - 0.95 (m, 1H), 0.65 - 0.85 (m, 2H), 0.40 - 0.55 (m, 2H), 0.00 - 0.15 (m, 2H).

### (Comparative Example 4)

### Synthesis of (1S,3aR,5aS,6R,11bS,11cS)-3-benzyl-14-(cyclopropylmethyl)-10-methoxy-11-(pyridine-3-yloxy)-2,3,3a,4,5,6,7,11c-octahydro-1H-6,11b-(epiminoethano)-1,5a-methanonaphtho[1,2-e]indole

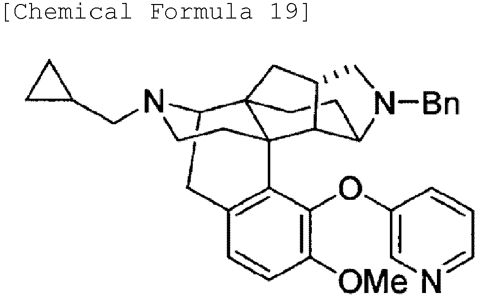

By a similar method to that in Example 1, using 3-iodopyridine as a halogenated heteroaryl, the title compound (0.14 g, yield: 80%) was obtained.

¹H-NMR (400 MHz, CDCl₃): δ8.15 - 8.25 (m, 2H), 7.15 - 7.35 (m, 6H), 7.02 (d, 2H, J = 8 Hz), 6.78 (d, 1H, J = 8 Hz), 3.72 (d, 1H, J = 13 Hz), 3.67 (s, 3H), 3.56 (d, 1H, J = 13 Hz), 2.95 - 3.15 (m, 6H), 2.65 - 2.80 (m, 2H), 2.47 (dd, 1H, J = 4, 12 Hz), 2.29 (d, 2H, J = 6 Hz), 2.00 (dt, 1H, J = 3, 13 Hz), 1.76 (dt, 1H, J = 5, 13 Hz), 1.55 - 1.70 (m, 2H), 1.15 - 1.30 (m, 2H), 1.00 - 1.10 (m, 1H), 0.90 - 1.00 (m, 1H), 0.70 - 0.85 (m, 2H), 0.45 - 0.55 (m, 2H), 0.00 - 0.15 (m, 2H).

### (Comparative Example 5)

### Synthesis of (1S,3aR,5aS,6R,11bS,11cS)-3-benzyl-14-(cyclopropylmethyl)-10-methoxy-11-(thiophene-3-yloxy)-2,3,3a,4,5,6,7,11c-octahydro-1H-6,11b-(epiminoethano)-1,5a-methanonaphtho[1,2-e]indole

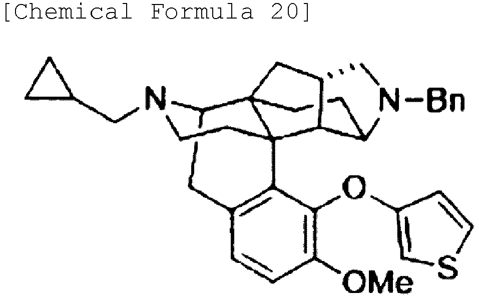

By a similar method to that in Example 1, using 3-bromothiophene as a halogenated heteroaryl, the title compound (0.081 g, yield: 71%) was obtained.

¹H-NMR (400 MHz, CDCl₃): δ7.10 - 7.40 (m, 6H), 6.96 (d, 1H, J = 8 Hz), 6.70 - 6.80 (m, 2H), 6.05 (dd, 1H, J = 2, 3 Hz), 3.72 (s, 3H), 3.70 (d, 1H, J = 13 Hz), 3.57 (d, 1H, J = 13 Hz), 2.90 - 3.20 (m, 6H), 2.60 - 2.80 (m, 2H), 2.40 - 2.50 (m, 1H), 2.28 (d, 2H, J = 6 Hz), 1.90 - 2.00 (m, 1H), 1.70 - 1.75 (m, 1H), 1.50 - 1.70 (m, 3H), 1.00 - 1.20 (m, 3H), 0.65 - 0.85 (m, 2H), 0.40 - 0.50 (m, 2H), 0.00 - 0.15 (m, 2H).

2. Production of ((1S,3aR,5aS,6R,11bR,11cS)-3-benzyl-14-(cyclopropylmethyl)-10-methoxy-2,3,3a,4,5,6,7,11c-octahydro-1H-6,11b-(epiminoethano)-1,5a-methanonaphtho[1,2-e]indole) in which the diaryl ether derivative produced in 1 above is used as a starting material

### (Example 4)

### Synthesis of (1S,3aR,5aS,6R,11bR,11cS)-3-benzyl-14-(cyclopropylmethyl)-10-methoxy-2,3,3a,4,5,6,7,11c-octahydro-1H-6,11b-(epiminoethano)-1,5a-methanonaphtho[1,2-e]indole dihydrochloride

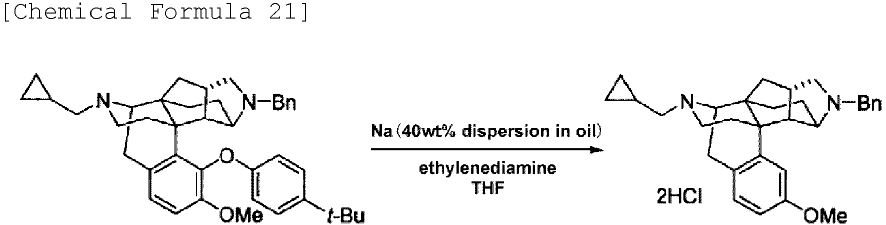

In a 200 mL round bottom flask, tetrahydrofuran (25 mL) was dissolved in ethylenediamine (25 mL) under a nitrogen atmosphere. Under an ice bath, metal sodium (5 g, 40 wt% dispersion in oil purchased from Alfa Aesar) was added to the obtained solution. After stirring for 10 minutes, into the obtained reaction liquid, a tetrahydrofuran (25 mL) solution of (1S,3aR,5aS,6R,11bS,11cS)-3-benzyl-11-(4-(tert-butyl)phenoxy)-14-(cyclopropylmethyl)-10-methoxy-2,3,3a,4,5,6,7,11c-octahydro-1H-6,11b-(epiminoethano)-1,5a-methanonaphtho[1,2-e]indole (5 g, 1 equivalent) obtained in the Example 1 was added dropwise with stirring. After 1 hour, the reaction was analyzed by MS and thin layer chromatography to confirm that the raw material remains. The reaction liquid was heated to room temperature. After 1 hour, the reaction was analyzed by MS and thin layer chromatography, and the disappearance of the raw material was confirmed.

Ethanol (25 mL) was added dropwise to the reaction liquid while stirring the reaction liquid in an ice bath, and a mixed liquid (25 mL) of ethanol/water = 4/1 was further added. Finally, water (25 mL) was added dropwise, and the resulting mixture was stirred for 1 hour to obtain a reaction liquid 1.

1 mL of the reaction liquid 1 was fractionated, and the production amounts of the target product and the by-product were checked by HPLC.

The remaining reaction liquid 1 was concentrated under reduced pressure, and a 6 N HCl aqueous solution was added to the obtained residue in an ice bath to adjust the pH of the liquid solution to 2. The obtained reaction liquid was washed with heptane (100 mL × 3 times), and the pH of the aqueous layer was adjusted to 12 with a 4 N NaOH aqueous solution. This aqueous layer was extracted with ethyl acetate (200 mL × 3 times). The combined organic layers were dried over anhydrous sodium sulfate, the anhydrous sodium sulfate was filtered off, and then the combined organic layers were concentrated under reduced pressure to obtain a title compound crude product.

The obtained crude product was dissolved in a mixed solvent of ethyl acetate/heptane = 1/4 (25 mL), and a 4 N hydrogen chloride/ethyl acetate solution (5 mL) was slowly added at 0 to 5°C with stirring to obtain a suspension.

The resulting solid was collected by filtration to obtain the title compound (4.18 g, 96%, purity: 94%).

The yield (%) of the target product obtained and the content (%) of the by-product corresponding to (b-1) are shown in Table 1. The production amounts of the target product ((1S,3aR,5aS,6R,11bR,11cS)-3-benzyl-14-(cyclopropylmethyl)-10-methoxy-2,3,3a,4,5,6,7,11c-octahydro-1H-6,11b-(epiminoethano)-1,5a-methanonaphtho[1,2-e]indole) and the by-product ((1S,3aR,5aS, 6R,11bS,11cS)-3-benzyl-14-(cyclopropylmethyl)-10-methoxy-2,3,3a,4,5,6,7,11c-octahydro-1H-6,11b-(epiminoethano)-1,5a-methanonaphtho[1,2-e]indole-11-ol) (hereinafter, referred to as a by-product A) in the reaction mixture were determined as an area percentage (%) of HPLC.

No by-product A was observed, and the yield of the target product was as high as 94%. This result shows that the dephenylation reaction was not observed, and the dephenoxylation reaction highly selectively occurred.

¹H-NMR (400 MHz, DMSO-d₆): δ10.40 - 10.65 (m, 1H), 9.29 (brs, 1H), 7.70 - 7.80 (m, 1H), 7.60 - 7.70 (m, 1H), 7.40 - 7.55 (m, 3H), 7.18 (d, 0.5H, J = 8 Hz), 7.13 (d, 0.5H, J = 8 Hz), 6.70 - 6.90 (m, 2H), 4.20 - 4.40 (m, 2H), 4.00 - 4.10 (m, 2H), 3.73 (s, 1.5H), 3.72 (s, 1.5H), 2.85 - 4.00 (m, 11H), 1.80 - 2.10 (m, 2H), 1.30 - 1.75 (m, 4H), 1.00 - 1.25 (m, 2H), 0.50 - 0.75 (m, 3H), 0.30 - 0.45 (m, 1H).

### (Reference Example 2)

Using the diaryl ether derivative produced in Reference Example 1 as a starting material, a dephenoxylation reaction was performed under Na-Benkeser reaction conditions by a method in accordance with Example 4 to produce the target product ((1S,3aR,5aS,6R,11bR,11cS)-3-benzyl-14-(cyclopropylmethyl)-10-methoxy-2,3,3a,4,5,6,7,11c-octahydro-1H-6,11b-(epiminoethano)-1,5a-methanonaphtho[1,2-e]indole). The yield (%) of the target product obtained and the content (%) of the by-product A are shown in Table 1.

The by-product A was produced by 5%, and the yield of the target product was 79%. This result shows that the dephenoxylation reaction was dominant, but the dephenylation reaction also occurred.

### (Example 5)

Using the diaryl ether derivative produced in Example 2 as a starting material, a dephenoxylation reaction was performed under Na-Benkeser reaction conditions by a method in accordance with Example 4 to produce the target product ((1S,3aR,5aS,6R,11bR,11cS)-3-benzyl-14-(cyclopropylmethyl)-10-methoxy-2,3,3a,4,5,6,7,11c-octahydro-1H-6,11b-(epiminoethano)-1,5a-methanonaphtho[1,2-e]indole). The yield (%) of the target product obtained and the content (%) of the by-product A are shown in Table 1.

The by-product A was produced by 1%, and the yield of the target product was as high as 89%. This result shows that though the dephenylation reaction slightly occurred, the dephenoxylation reaction occurred more selectively than in Reference Example 2.

### (Example 6)

Using the diaryl ether derivative produced in Example 3 as a starting material, a dephenoxylation reaction was performed under Na-Benkeser reaction conditions by a method in accordance with Example 4 to produce the target product ((1S,3aR,5aS,6R,11bR,11cS)-3-benzyl-14-(cyclopropylmethyl)-10-methoxy-2,3,3a,4,5,6,7,11c-octahydro-1H-6,11b-(epiminoethano)-1,5a-methanonaphtho[1,2-e]indole). The yield (%) of the target product obtained and the content (%) of the by-product A are shown in Table 1.

No by-product A was observed, and the yield of the target product was as high as 93%. This result shows that the dephenylation reaction was not observed, and the dephenoxylation reaction highly selectively occurred.

### (Comparative Example 6)

Using the diaryl ether derivative produced in Comparative Example 1 as a starting material, a dephenoxylation reaction was performed under Na-Benkeser reaction conditions by a method in accordance with Example 4 to produce the target product ((1S,3aR,5aS,6R,11bR,11cS)-3-benzyl-14-(cyclopropylmethyl)-10-methoxy-2,3,3a,4,5,6,7,11c-octahydro-1H-6,11b-(epiminoethano)-1,5a-methanonaphtho[1,2-e]indole). The yield (%) of the target product obtained and the content (%) of the by-product A are shown in Table 1.

The by-product A was produced by 3%, and the yield of the target product was 30%. This result shows that the dephenoxylation reaction was dominant, but the dephenylation reaction also occurred, and the selectivity of the dephenoxylation reaction was reduced as compared with Reference Example 2.

### (Comparative Example 7)

Using the diaryl ether derivative produced in Comparative Example 2 as a starting material, a dephenoxylation reaction was performed under Na-Benkeser reaction conditions by a method in accordance with Example 4 to produce the target product ((1S,3aR,5aS,6R,11bR,11cS)-3-benzyl-14-(cyclopropylmethyl)-10-methoxy-2,3,3a,4,5,6,7,11c-octahydro-1H-6,11b-(epiminoethano)-1,5a-methanonaphtho[1,2-e]indole). The yield (%) of the target product obtained and the content (%) of the by-product A are shown in Table 1.

The by-product A was produced by 6%, and the yield of the target product was 42%. This result shows that the dephenoxylation reaction was dominant, but the dephenylation reaction also occurred, and the selectivity of the dephenoxylation reaction was reduced as compared with Reference Example 2.

### (Comparative Example 8)

Using the diaryl ether derivative produced in Comparative Example 3 as a starting material, a dephenoxylation reaction was performed under Na-Benkeser reaction conditions by a method in accordance with Example 4 to produce the target product ((1S,3aR,5aS,6R,11bR,11cS)-3-benzyl-14-(cyclopropylmethyl)-10-methoxy-2,3,3a,4,5,6,7,11c-octahydro-1H-6,11b-(epiminoethano)-1,5a-methanonaphtho[1,2-e]indole). The yield (%) of the target product obtained and the content (%) of the by-product A are shown in Table 1.

The by-product A was produced by 35%, and the yield of the target product was 2%. This result shows that the dephenylation reaction occurred more dominantly than the dephenoxylation reaction.

### (Comparative Example 9)

Using the diaryl ether derivative produced in Comparative Example 4 as a starting material, a dephenoxylation reaction was performed under Na-Benkeser reaction conditions by a method in accordance with Example 4 to produce the target product ((1S,3aR,5aS,6R,11bR,11cS)-3-benzyl-14-(cyclopropylmethyl)-10-methoxy-2,3,3a,4,5,6,7,11c-octahydro-1H-6,11b-(epiminoethano)-1,5a-methanonaphtho[1,2-e]indole). The yield (%) of the target product obtained and the content (%) of the by-product A are shown in Table 1.

The by-product A was produced by 10%, and the yield of the target product was 76%. This result shows that the dephenoxylation reaction was dominant, but the dephenylation reaction also occurred, and the selectivity of the dephenoxylation reaction was reduced as compared with Reference Example 2.

### (Comparative Example 10)

Using the diaryl ether derivative produced in Comparative Example 5 as a starting material, a dephenoxylation reaction was performed under Na-Benkeser reaction conditions by a method in accordance with Example 4 to produce the target product ((1S,3aR,5aS,6R,11bR,11cS)-3-benzyl-14-(cyclopropylmethyl)-10-methoxy-2,3,3a,4,5,6,7,11c-octahydro-1H-6,11b-(epiminoethano)-1,5a-methanonaphtho[1,2-e]indole). The yield (%) of the target product obtained and the content (%) of the by-product A are shown in Table 1.

The by-product A was produced by 3%, and the yield of the target product was 67%. This result shows that the dephenoxylation reaction was dominant, but the dephenylation reaction also occurred, and the yield of the dephenoxylation reaction was lower than that of Reference Example 2.

The test results of Reference Example 2, Examples 4 to 6, and Comparative Examples 6 to 10 described above are shown in Table 1 below.

**[Table 1]**

| Test | Starting material | By-product A (%) | Target product (%) |
|---|---|---|---|
| Reference Example 2 | 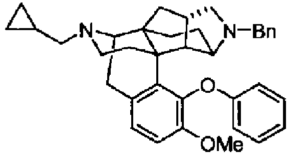 | 5 | 79 |
| Example 4 | 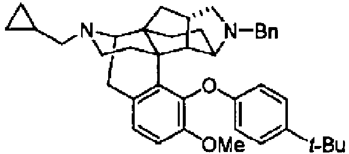 | none | 94 |
| Example 5 | 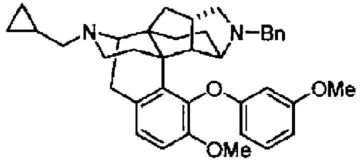 | 1 | 89 |
| Example 6 | 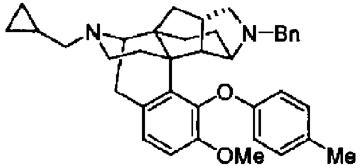 | none | 93 |
| Comparative Example 6 | 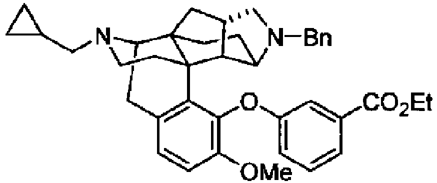 | 3 | 30 |
| Comparative Example 7 | 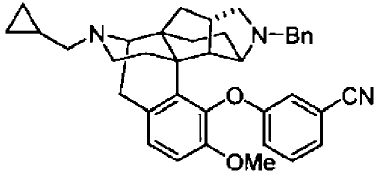 | 6 | 42 |
| Comparative Example 8 | 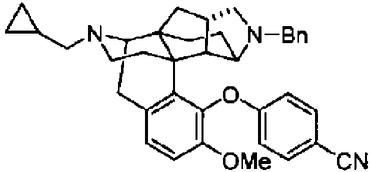 | 35 | 2 |
| Comparative Example 9 | 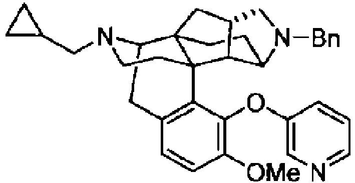 | 10 | 76 |
| Comparative Example 10 | 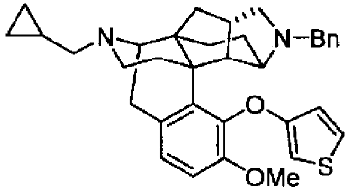 | 3 | 67 |

### Industrial Applicability

According to the present invention, a method for highly selectively producing a morphinan derivative from a diaryl ether derivative by suppressing production of a phenol form as a by-product, the method being applicable to an industrial production method can be provided.

## Claims

1. A method for producing a morphinan derivative represented by General Formula (II) below: 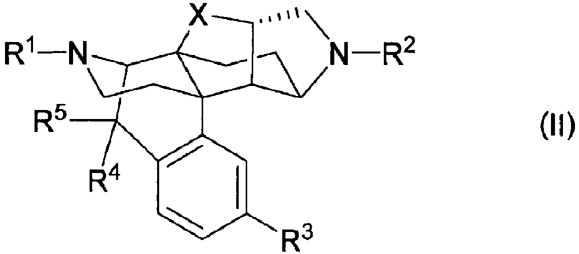
wherein R¹ represents a hydrogen atom, a C₁₋₁₀ alkyl group optionally having a substituent, a C₃₋₆ cycloalkyl group optionally having a substituent, a C₂₋₆ alkenyl group optionally having a substituent, a C₆₋₁₀ aryl group optionally having a substituent, or a heteroaryl group optionally having a substituent,
R² represents an amino protecting group selected from a carbamate protecting group, a sulfonamide protecting group, an acyl protecting group, and a C₇₋₁₉ aralkyl group,
R³ represents a hydrogen atom, a halogen atom, or a C₁₋₁₀ alkoxy group optionally having a substituent,
R⁴ and R⁵ are same or different and represent a hydrogen atom or a hydroxy group, and
X represents O or CH₂,
comprising the step of:
allowing metal sodium and ethylenediamine to act on a morphinan derivative represented by
General Formula (I) below: 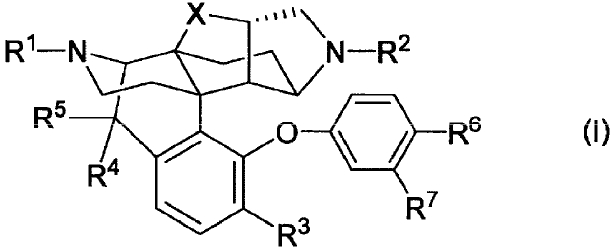
wherein R¹ to R⁵ and X have a meaning same as above,
R⁶ and R⁷ are same or different and represent a hydrogen atom or an electron-donating group,
wherein R⁶ and R⁷ are not simultaneously a hydrogen atom, and
in presence of an auxiliary solvent.

2. The method for producing a morphinan derivative according to claim 1, wherein R² is a benzyl group.

3. The method for producing a morphinan derivative according to claim 1 or 2, wherein R³ is a methoxy group.

4. The method for producing a morphinan derivative according to any one of claims 1 to 3, wherein R⁶ is a C₁₋₁₀ alkyl group, OR⁸ (R⁸ represents a hydrogen atom or a C₁₋₁₀ alkyl group optionally having a substituent), or NR⁹R¹⁰ (R⁹ and R¹⁰ are same or different and represent a hydrogen atom or a C₁₋₁₀ alkyl group optionally having a substituent).

5. The method for producing a morphinan derivative according to any one of claims 1 to 3, wherein R⁶ is a hydrogen atom.

6. The method for producing a morphinan derivative according to any one of claims 1 to 4, wherein R⁷ is a hydrogen atom.

7. The method for producing a morphinan derivative according to any one of claims 1 to 5, wherein R⁷ is a C₁₋₁₀ alkyl group optionally having a substituent, OR¹¹ (R¹¹ represents a hydrogen atom or a C₁₋₁₀ alkyl group optionally having a substituent), or NR¹²R¹³ (R¹² and R¹³ are same or different and represent a hydrogen atom or a C₁₋₁₀ alkyl group optionally having a substituent).

8. The method for producing a morphinan derivative according to any one of claims 1 to 7, wherein the metal sodium is used in an amount of 1 to 10 equivalents relative to the morphinan derivative of the General Formula (I).

9. The method for producing a morphinan derivative according to any one of claims 1 to 8, wherein the metal sodium is a metal sodium dispersion.

10. The method for producing a morphinan derivative according to any one of claims 1 to 9, wherein the auxiliary solvent is an aromatic hydrocarbon solvent or an ether solvent.

11. The method for producing a morphinan derivative according to any one of claims 1 to 10, wherein a reaction temperature is -30°C to 150°C.

12. The method for producing a morphinan derivative according to any one of claims 1 to 11, wherein a reaction temperature is 0°C to 30°C.

13. A morphinan derivative represented by
General Formula (I) below: 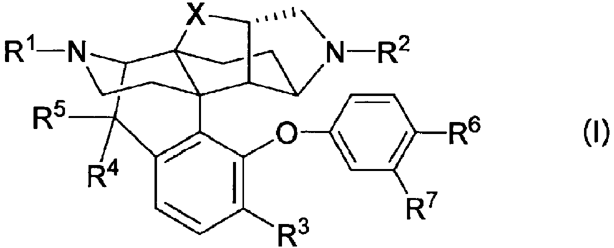
wherein R¹ represents a hydrogen atom, a C₁₋₁₀ alkyl group optionally having a substituent, a C₃₋₆ cycloalkyl group optionally having a substituent, a C₂₋₆ alkenyl group optionally having a substituent, a C₆₋₁₀ aryl group optionally having a substituent, or a heteroaryl group optionally having a substituent,
R² represents an amino protecting group selected from a carbamate protecting group, a sulfonamide protecting group, an acyl protecting group, and a C₇₋₁₉ aralkyl group,
R³ represents a hydrogen atom, a halogen atom, or a C₁₋₁₀ alkoxy group optionally having a substituent,
R⁴ and R⁵ are same or different and represent a hydrogen atom or a hydroxy group,
R⁶ and R⁷ are same or different and represent a hydrogen atom or an electron-donating group,
wherein R⁶ and R⁷ are not simultaneously a hydrogen atom, and
X represents O or CH₂.

14. The morphinan derivative according to claim 13, wherein R² is a benzyl group.

15. The morphinan derivative according to claim 13 or 14, wherein R³ is a methoxy group.

16. The morphinan derivative according to any one of claims 13 to 15, wherein R⁶ is a C₁₋₁₀ alkyl group, OR⁸ (R⁸ represents a hydrogen atom or a C₁₋₁₀ alkyl group optionally having a substituent), or NR⁹R¹⁰ (R⁹ and R¹⁰ are same or different and represent a hydrogen atom or a C₁₋₁₀ alkyl group optionally having a substituent).

17. The morphinan derivative according to any one of claims 13 to 15, wherein R⁶ is a hydrogen atom.

18. The morphinan derivative according to any one of claims 13 to 15, wherein R⁷ is a hydrogen atom.

19. The morphinan derivative according to any one of claims 13 to 17, wherein R⁷ is a C₁₋₁₀ alkyl group optionally having a substituent, OR¹¹ (R¹¹ represents a hydrogen atom or a C₁₋₁₀ alkyl group optionally having a substituent), or NR¹²R¹³ (R¹² and R¹³ are same or different and represent a hydrogen atom or a C₁₋₁₀ alkyl group optionally having a substituent).

20. The morphinan derivative according to claim 13, wherein the derivative is selected from
(1S,3aR,5aS,6R,11bS,11cS)-3-benzyl-11-(4-(tert-butyl)phenoxy)-14-(cyclopropylmethyl)-10-methoxy-2,3,3a,4,5,6,7,11c-octahydro-1H-6,11b-(epiminoethano)-1,5a-methanonaphtho[1,2-e]indole,
(1S,3aR,5aS,6R,11bS,11cS)-3-benzyl-14-(cyclopropylmethyl)-10-methoxy-11-(3-methoxyphenoxy)-2,3,3a,4,5,6,7,11c-octahydro-1H-6,11b-(epiminoethano)-1,5a-methanonaphtho[1,2-e]indole, and
(1S,3aR,5aS,6R,11bS,11cS)-3-benzyl-14-(cyclopropylmethyl)-10-methoxy-11-(p-toluyloxy)-2,3,3a,4,5,6,7,11c-octahydro-1H-6,11b-(epiminoethano)-1,5a-methanonaphtho[1,2-e]indole.

## Patentansprüche

1. Ein Verfahren zur Herstellung von Morphinan-Derivaten, dargestellt durch die unten stehende allgemeine Formel (II): 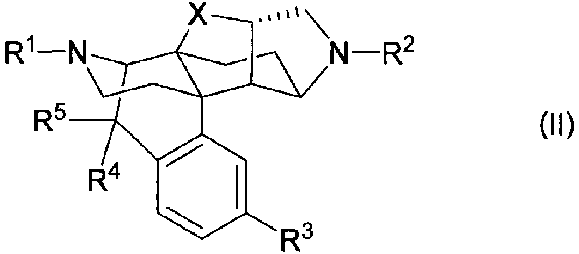
wobei R¹ ein Wasserstoffatom, eine C₁₋₁₀-Alkylgruppe, die einen Substituenten aufweisen kann, eine C₃₋₆-Cycloalkylgruppe, die einen Substituenten aufweisen kann, eine C₂₋₆-Alkenylgruppe, die einen Substituenten aufweisen kann, eine C₆₋₁₀-Arylgruppe, die einen Substituenten aufweisen kann, oder eine Heteroarylgruppe, die einen Substituenten aufweisen kann, darstellt,
R² eine Aminoschutzgruppe darstellt ausgewählt aus einer Carbamat-Schutzgruppe, einer Sulfonamid-Schutzgruppe, einer Acyl-Schutzgruppe und einer C₇₋₁₉-Aralkylgruppe,
R³ ein Wasserstoffatom, ein Halogenatom, oder eine C₁₋₁₀-Alkoxygruppe, die einen Substituenten aufweisen kann, darstellt,
R⁴ und R⁵ gleich oder verschieden sind und ein Wasserstoffatom oder eine Hydroxylgruppe darstellen, und
X O oder CH₂ darstellt,
umfassend den Schritt:
wobei metallisches Natrium und Ethylendiamin auf ein Morphinan-Derivat einwirken können, das durch die folgende allgemeine Formel (I) unten dargestellt wird: 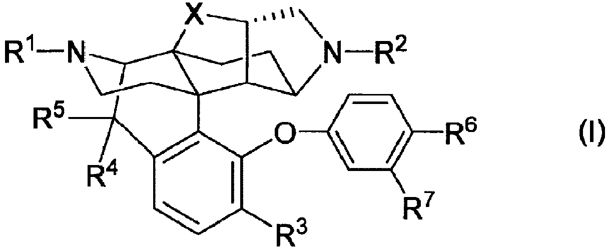
wobei R¹ bis R⁵ und X die gleiche Bedeutung haben wie oben,
R⁶ und R⁷ gleich oder verschieden sind und ein Wasserstoffatom oder eine Elektronendonorgruppe darstellen,
wobei R⁶ und R⁷ nicht gleichzeitig ein Wasserstoffatom darstellen, und
in Anwesenheit eines Hilfslösungsmittels.

2. Das Verfahren zur Herstellung eines Morphinan-Derivats gemäß Anspruch 1, wobei R² eine Benzylgruppe ist.

3. Das Verfahren zur Herstellung eines Morphinan-Derivats gemäß Anspruch 1 oder 2, wobei R³ eine Methoxygruppe ist.

4. Das Verfahren zur Herstellung eines Morphinan-Derivats gemäß irgend einem der Ansprüche 1 bis 3, wobei R⁶ eine C₁₋₁₀-Alkylgruppe, OR⁸ (wobei R⁸ ein Wasserstoffatom oder eine C₁₋₁₀ -Alkylgruppe darstellt, die einen Substituenten aufweisen kann), oder NR⁹R¹⁰ (wobei R⁹ und R¹⁰ gleich oder verschieden sind und ein Wasserstoffatom oder eine C₁₋₁₀-Alkylgruppe darstellen, die einen Substituenten aufweisen kann) darstellt.

5. Das Verfahren zur Herstellung eines Morphinan-Derivats gemäß irgend einem der Ansprüche 1 bis 3, wobei R⁶ ein Wasserstoffatom ist.

6. Das Verfahren zur Herstellung eines Morphinan-Derivats gemäß irgend einem der Ansprüche 1 bis 4, wobei R⁷ ein Wasserstoffatom ist.

7. Das Verfahren zur Herstellung eines Morphinan-Derivats gemäß irgend einem der Ansprüche 1 bis 5, wobei R⁷ eine C₁₋₁₀-Alkylgruppe, die einen Substituenten aufweisen kann, OR¹¹ (wobei R¹¹ ein Wasserstoffatom oder eine C₁₋₁₀-Alkylgruppe darstellt, die einen Substiuenten aufweisen kann), oder NR¹²R¹³ (wobei R¹² und R¹³ gleich oder verschieden sind und ein Wasserstoffatom oder eine C₁₋₁₀-Alkylgruppe darstellen, die einen Substituenten aufweisen kann) darstellt.

8. Das Verfahren zur Herstellung eines Morphinan-Derivats gemäß irgend einem der Ansprüche 1 bis 7, wobei das metallische Natrium in einer Menge von 1 bis 10 Äquivalenten bezogen auf das Morphinan-Derivat der allgemeinen Formel (I) eingesetzt wird.

9. Das Verfahren zur Herstellung eines Morphinan-Derivats gemäß irgend einem der Ansprüche 1 bis 8, wobei das metallische Natrium eine metallische Natriumdispersion ist.

10. Das Verfahren zur Herstellung eines Morphinan-Derivats gemäß irgend einem der Ansprüche 1 bis 9, wobei das Hilfslösungsmittel ein aromatisches Kohlenwasserstofflösungsmittel oder ein Etherlösungsmittel ist.

11. Das Verfahren zur Herstellung eines Morphinan-Derivats gemäß irgend einem der Ansprüche 1 bis 10, wobei die Reaktionstemperatur -30°C bis 150°C beträgt.

12. Das Verfahren zur Herstellung eines Morphinan-Derivats gemäß irgend einem der Ansprüche 1 bis 11, wobei die Reaktionstemperatur 0°C bis 30°C beträgt.

13. Ein Morphinan-Derivat, das durch die folgende allgemeine Formel (I) dargestellt wird: 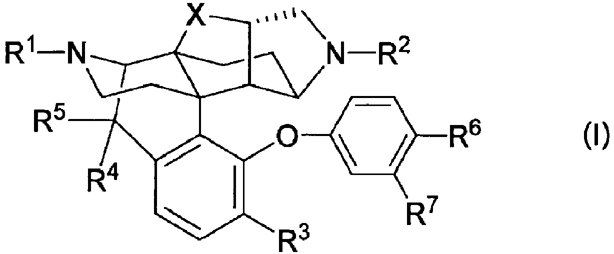 wobei R¹ ein Wasserstoffatom, eine C₁₋₁₀-Alkylgruppe, die einen Substituenten aufweisen kann, eine C₃₋₆-Cycloalkylgruppe, die einen Substituenten aufweisen kann, eine C₂₋₆-Alkenylgruppe, die einen Substituenten aufweisen kann, eine C₆₋₁₀-Arylgruppe, die einen Substituenten aufweisen kann, oder eine Heteroarylgruppe, die einen Substituenten aufweisen kann, darstellt,
R² eine Aminoschutzgruppe darstellt ausgewählt aus einer Carbamat-Schutzgruppe, einer Sulfonamid-Schutzgruppe, einer Acyl-Schutzgruppe, und einer C₇₋₁₉-Aralkylgruppe,
R³ ein Wasserstoffatom, ein Halogenatom, oder eine C₁₋₁₀-Alkoxygruppe, die einen Substituenten aufweisen kann, darstellt,
R⁴ und R⁵ gleich oder verschieden sind und ein Wasserstoffatom oder eine Hydroxylgruppe darstellen,
R⁶ und R⁷ gleich oder verschieden sind und ein Wasserstoffatom oder eine Elektronendonorgruppe darstellen,
wobei R⁶ und R⁷ nicht gleichzeitig ein Wasserstoffatom darstellen, und
X O oder CH₂ darstellt.

14. Das Morphinan-Derivat gemäß Anspruch 13, wobei R² eine Benzylgruppe ist.

15. Das Morphinan-Derivat gemäß Anspruch 13 oder 14, wobei R³ eine Methoxygruppe ist.

16. Das Morphinan-Derivat gemäß irgend einem der Ansprüche 13 bis 15, wobei R⁶ eine C₁₋₁₀-Alkylgruppe, OR⁸ (wobei R⁸ ein Wasserstoffatom oder eine C₁₋₁₀-Alkylgruppe darstellt, die einen Substituenten aufweisen kann), oder NR⁹R¹⁰ (wobei R⁹ und R¹⁰ gleich oder verschieden sind und ein Wasserstoffatom oder eine C₁₋₁₀-Alkylgruppe darstellen, die einen Substituenten aufweisen kann), darstellt.

17. Das Morphinan-Derivat gemäß irgend einem der Ansprüche 13 bis 15, wobei R⁶ ein Wasserstoffatom ist.

18. Das Morphinan-Derivat gemäß irgend einem der Ansprüche 13 bis 15, wobei R⁷ ein Wasserstoffatom ist.

19. Das Morphinan-Derivat gemäß irgend einem der Ansprüche 13 bis 17, wobei R⁷ eine C₁₋₁₀-Alkylgruppe, die einen Substituenten aufweisen kann, OR¹¹ (wobei R¹¹ ein Wasserstoffatom oder eine C₁₋₁₀-Alkylgruppe darstellt, die einen Substituenten aufweisen kann), oder NR¹²R¹³ (wobei R¹² und R¹³ gleich oder verschieden sind und ein Wasserstoffatom oder eine C₁₋₁₀-Alkylgruppe darstellen, die einen Substituenten aufweisen kann) darstellt.

20. Das Morphinan-Derivat gemäß Anspruch 13, wobei das Derivativ ausgewählt ist aus
(1S,3aR,5aS,6R,11bS,11cS)-3-Benzyl-11-(4-(tert-butyl)phenoxy)-14-(cyclopropylmethyl)-10-methoxy-2,3,3a,4,5,6,7,11c-octahydro-1H-6,11b-(epiminoethano)-1,5a-methanonaphtho[1,2-e]indol,
(1S,3aR,5aS,6R,11bS,11cS)-3-Benzyl-14-(cyclopropylmethyl)-10-methoxy-11-(3-methoxyphenoxy)-2,3,3a,4,5,6,7,11c-octahydro-1H-6,11b-(epiminoethano)-1,5a-methanonaphtho[1,2-e]indol, und
(1S,3aR,5aS,6R,11bS,11cS)-3-Benzyl-14-(cyclopropylmethyl)-10-methoxy-11-(p-toluyloxy)-2,3,3a,4,5,6,7,11c-octahydro-1H-6,11b-(epiminoethano)-1,5a-methanonaphtho[1,2-e]indol.

## Revendications

1. Procédé de préparation d'un dérivé de morphinane représenté par la formule générale (II) ci-dessous : 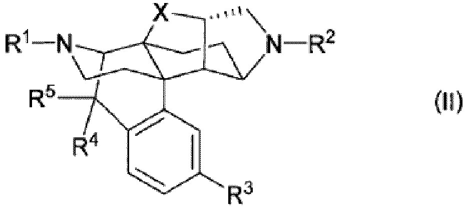
où R¹ représente un atome d'hydrogène, un groupe alkyle en C₁₋₁₀ comportant éventuellement un substituant, un groupe cycloalkyle en C₃₋₆ comportant éventuellement un substituant, un groupe alcényle en C₂₋₆ comportant éventuellement un substituant, un groupe aryle en C₆₋₁₀ comportant éventuellement un substituant, ou un groupe hétéroaryle comportant éventuellement un substituant,
R² représente un groupe protecteur d'amino choisi parmi un groupe protecteur carbamate, un groupe protecteur sulfonamide, un groupe protecteur acyle et un groupe aralkyle en C₇₋₁₉,
R³ représente un atome d'hydrogène, un atome d'halogène ou un groupe alcoxy en C₁₋₁₀ comportant éventuellement un substituant,
R⁴ et R⁵ sont identiques ou différents et représentent un atome d'hydrogène ou un groupe hydroxy, et
X représente O ou CH₂,
comprenant l'étape consistant à :
permettre au sodium métallique et au éthylènediamine d'agir sur un dérivé de morphinan représenté par
Formule Générale (I) ci-dessous : 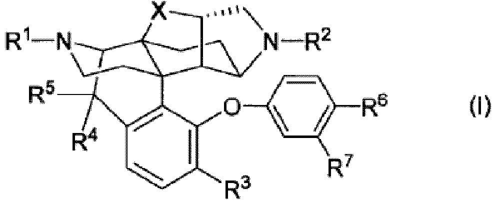
où R¹ à R⁵ et X ont la même signification que ci-dessus,
R⁶ et R⁷ sont identiques ou différents et représentent un atome d'hydrogène ou un groupe donneur d'électrons,
où R⁶ et R⁷ ne sont pas simultanément des atomes d'hydrogène, et
en présence d'un solvant auxiliaire.

2. Procédé de préparation d'un dérivé de morphinane selon la revendication 1, dans lequel R² est un groupe benzyle.

3. Procédé de préparation d'un dérivé de morphinane selon la revendication 1 ou 2, dans lequel R³ est un groupe méthoxy.

4. Procédé de préparation d'un dérivé de morphinane selon l'une quelconque des revendications 1 à 3, dans lequel R⁶ est un groupe alkyle en C₁₋₁₀, OR⁸ (R⁸ représente un atome d'hydrogène ou un groupe alkyle en C₁₋₁₀ comportant éventuellement un substituant), ou NR⁹R¹⁰ (R⁹ et R¹⁰ sont identiques ou différents et représentent un atome d'hydrogène ou un groupe alkyle en C₁₋₁₀ comportant éventuellement un substituant).

5. Procédé de préparation d'un dérivé de morphinane selon l'une quelconque des revendications 1 à 3, dans lequel R⁶ est un atome d'hydrogène.

6. Procédé de préparation d'un dérivé de morphinane selon l'une quelconque des revendications 1 à 4, dans lequel R⁷ est un atome d'hydrogène.

7. Procédé de préparation d'un dérivé de morphinane selon l'une quelconque des revendications 1 à 5, dans lequel R⁷ est un groupe alkyle en C₁₋₁₀ pouvant éventuellement comporter un substituant, OR¹¹ (R¹¹ représente un atome d'hydrogène ou un groupe alkyle en C₁₋₁₀ pouvant éventuellement comporter un substituant), ou NR¹²R¹³ (R¹² et R¹³ sont identiques ou différents et représentent un atome d'hydrogène ou un groupe alkyle en C₁₋₁₀ comportant éventuellement un substituant).

8. Procédé de préparation d'un dérivé de morphinane selon l'une quelconque des revendications 1 à 7, dans lequel le sodium métallique est utilisé en une quantité de 1 à 10 équivalents par rapport au dérivé de morphinane de formule générale (I).

9. Procédé de préparation d'un dérivé de morphinane selon l'une quelconque des revendications 1 à 8, dans lequel le sodium métallique est une dispersion de sodium métallique.

10. Procédé de préparation d'un dérivé de morphinane selon l'une quelconque des revendications 1 à 9, dans lequel le solvant auxiliaire est un solvant à base d'hydrocarbure aromatique ou un solvant à base d'éther.

11. Procédé de préparation d'un dérivé de morphinane selon l'une quelconque des revendications 1 à 10, dans lequel la température de réaction est comprise entre -30 °C et 150 °C.

12. Procédé de préparation d'un dérivé de morphinane selon l'une quelconque des revendications 1 à 11, dans lequel la température de réaction est comprise entre 0 °C et 30 °C.

13. Dérivé de morphinane représenté par la formule générale (I) ci-dessous : 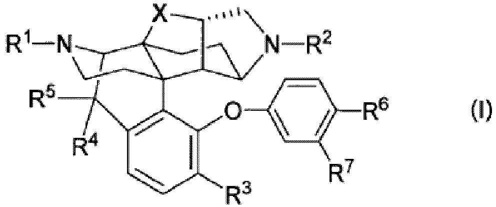
où R¹ représente un atome d'hydrogène, un groupe alkyle en C₁₋₁₀ comportant éventuellement un substituant, un groupe cycloalkyle en C₃₋₆ comportant éventuellement un substituant, un groupe alcényle en C₂₋₆ comportant éventuellement un substituant, un groupe aryle en C₆₋₁₀ comportant éventuellement un substituant, ou un groupe hétéroaryle comportant éventuellement un substituant,
R² représente un groupe protecteur d'amino choisi parmi un groupe protecteur carbamate, un groupe protecteur sulfonamide, un groupe protecteur acyle et un groupe aralkyle en C₇₋₁₉,
R³ représente un atome d'hydrogène, un atome d'halogène ou un groupe alcoxy en C₁₋₁₀ comportant éventuellement un substituant,
R⁴ et R⁵ sont identiques ou différents et représentent un atome d'hydrogène ou un groupe hydroxy,
R⁶ et R⁷ sont identiques ou différents et représentent un atome d'hydrogène ou un groupe donneur d'électrons,
où R⁶ et R⁷ ne sont pas simultanément des atomes d'hydrogène, et
X représente O ou CH₂.

14. Dérivé de morphinane selon la revendication 13, dans lequel R² est un groupe benzyle.

15. Dérivé de morphinane selon la revendication 13 ou 14, dans lequel R³ est un groupe méthoxy.

16. Dérivé de morphinane selon l'une quelconque des revendications 13 à 15, dans lequel R⁶ est un groupe alkyle en C₁₋₁₀, OR⁸ (R⁸ représente un atome d'hydrogène ou un groupe alkyle en C₁₋₁₀ comportant éventuellement un substituant), ou NR⁹R¹⁰ (R⁹ et R¹⁰ sont identiques ou différents et représentent un atome d'hydrogène ou un groupe alkyle en C₁₋₁₀ comportant éventuellement un substituant).

17. Dérivé de morphinane selon l'une quelconque des revendications 13 à 15, dans lequel R⁶ est un atome d'hydrogène.

18. Dérivé de morphinane selon l'une quelconque des revendications 13 à 15, dans lequel R⁷ est un atome d'hydrogène.

19. Dérivé de morphinane selon l'une quelconque des revendications 13 à 17, dans lequel R⁷ est un groupe alkyle en C₁₋₁₀ comportant éventuellement un substituant, OR¹¹ (R¹¹ représente un atome d'hydrogène ou un groupe alkyle en C₁₋₁₀ comportant éventuellement un substituant), ou NR¹² R¹³ (R¹² et R¹³ sont identiques ou différents et représentent un atome d'hydrogène ou un groupe alkyle en C₁₋₁₀ comportant éventuellement un substituant).

20. Dérivé de morphinane selon la revendication 13, dans lequel le dérivé est choisi parmi le (1S,3aR,5aS,6R,11bS,11cS)-3-benzyl-11-(4-(tert-butyl)phénoxy)-14-(cyclopropylméthyl)-10-méthoxy-2,3,3a,4,5,6,7,11c-octahydro-1H-6,11b-(épiminoéthano)-1,5a-méthanonaphto[1,2-e]indole, (1S,3aR,5aS,6R,11bS,11cS)-3-benzyl-14-(cyclopropylméthyl)-10-méthoxy-11-(3-méthoxyphénoxy)-2,3,3a,4,5,6,7,11c-octahydro-1H-6,11b-(épiminoéthano)-1,5a-méthanonaphto[1,2-e]indole, et
(1S,3aR,5aS,6R,11bS,11cS)-3-benzyl-14-(cyclopropylméthyl)-10-méthoxy-11-(p-toluyloxy)-2,3,3a,4,5,6,7,11c-octahydro-1H-6,11b-(épiminoéthano)-1,5a-méthanonaphto[1,2-e]indole.
